# EUROPEAN PATENT SPECIFICATION

(11) **EP 4 381 094 B1**
(45) Date of publication and mention of the grant of the patent: **02.07.2025**
(21) Application number: 22750923.9
(22) Date of filing: 03.08.2022
(51) Int. Cl.: C12Q 1/6816

(54) **ARGONAUTE-BASED NUCLEIC ACID DETECTION SYSTEM**
NUKLEINSÄUREDETEKTIONSSYSTEM AUF ARGONAUTEN-BASIS
SYSTÈME DE DÉTECTION D'ACIDES NUCLÉIQUES À BASE D'ARGONAUTE

(30) Priority: 03.08.2021 EP 21189448
(43) Date of publication of application: 12.06.2024
(73) Proprietor: Wageningen Universiteit, 6708 PB Wageningen (NL)
(72) Inventor: SWARTS, Daniël Christianus, 6721 VT Bennekom (NL); KOOPAL, Balwina, 6701 BB Wageningen (NL); POTOCNIK, Ana, 6707 AA Wageningen (NL)
(74) Representative: V.O.
(86) International application number: PCT/NL2022/050457
(87) International publication number: WO 2023/014222

(56) References cited:
- HEGGE JORRIT W ET AL: "Prokaryotic Argonaute proteins: novel genome-editing tools?", NATURE REVIEWS MICROBIOLOGY,, vol. 16, no. 1, 1 January 2018 (2018-01-01), pages 5 - 11, XP002780880, DOI: 10.1038/NRMICRO.2017.73
- DAAN C SWARTS ET AL: "The evolutionary journey of Argonaute proteins", NATURE STRUCTURAL & MOLECULAR BIOLOGY, vol. 21, no. 9, 5 September 2014 (2014-09-05), New York, pages 743 - 753, XP055287457, ISSN: 1545-9993, DOI: 10.1038/nsmb.2879
- JIN SHUJUAN ET AL: "Argonaute proteins: structures and their endonuclease activity", MOLECULAR BIOLOGY REPORTS, SPRINGER NETHERLANDS, NL, vol. 48, no. 5, 1 May 2021 (2021-05-01), pages 4837 - 4849, XP037498667, ISSN: 0301-4851, [retrieved on 20210611], DOI: 10.1007/S11033-021-06476-W
- MAKAROVA KIRA S ET AL: "Prokaryotic homologs of Argonaute proteins are predicted to function as key components of a novel system of defense against mobile genetic elements", BIOLOGY DIRECT, BIOMED CENTRAL, vol. 4, no. 1, 25 August 2009 (2009-08-25), pages 29, XP021059840, ISSN: 1745-6150, DOI: 10.1186/1745-6150-4-29
- RYAZANSKY SERGEI ET AL: "The Expanded Universe of Prokaryotic Argonaute Proteins", vol. 9, no. 6, 21 December 2018 (2018-12-21), US, XP055880624, ISSN: 2161-2129, Retrieved from the Internet <URL:https://journals.asm.org/doi/pdf/10.1128/mBio.01935-18> DOI: 10.1128/mBio.01935-18
- KIM SEUNG-YOON ET AL: "Argonaute system of Kordia jejudonensis is a heterodimeric nucleic acid-guided nuclease", BIOCHEMICAL AND BIOPHYSICAL RESEARCH COMMUNICATIONS, ELSEVIER, AMSTERDAM NL, vol. 525, no. 3, 4 March 2020 (2020-03-04), pages 755 - 758, XP086121284, ISSN: 0006-291X, [retrieved on 20200304], DOI: 10.1016/J.BBRC.2020.02.145
- ESSUMAN KOW ET AL: "TIR Domain Proteins Are an Ancient Family of NAD+-Consuming Enzymes", CURRENT BIOLOGY, CURRENT SCIENCE, GB, vol. 28, no. 3, 25 January 2018 (2018-01-25), pages 421, XP085347131, ISSN: 0960-9822, DOI: 10.1016/J.CUB.2017.12.024
- OFIR GAL ET AL: "Antiviral activity of bacterial TIR domains via signaling molecules that trigger cell death", BIORXIV, 6 January 2021 (2021-01-06), XP055881710, Retrieved from the Internet <URL:https://www.biorxiv.org/content/10.1101/2021.01.06.425286v1.full.pdf> [retrieved on 20220120], DOI: 10.1101/2021.01.06.425286

## Description

FIELD: The invention described herein is directed to a short prokaryotic Argonaute-related nucleic acid detection system and its broad use in diagnostic applications.

### BACKGROUND TO THE INVENTION

Argonaute proteins recruit small (~14-30 nt) single stranded oligonucleotides (the guide) to bind a complementary single stranded oligonucleotides (the target). All Eukaryotic Argonaute proteins (eAgo proteins) characterized to date use RNA guides to bind and/or cleave RNA targets (Ma et al., 2018. Brief Funct Genomics 17: 191-197). This process underlies RNA interference (RNAi). Prokaryotes encode homologs of eAgo proteins (pAgo proteins), but lack other proteins required for RNAi (Swarts et al., 2014. Nat Struct Mol Biol 21: 743-753). Based on the genetic co-localization with genes involved in prokaryotic immunity, it was hypothesized that pAgo proteins protect their host against invading nucleic acids (Makarova et al., 2009. Biol Direct 4: 29). Indeed, various pAgo proteins interfere with invading nucleic acids such as plasmids (Olovnikov et al., 2013. Mol Cell 51: 594-605; Swarts et al., 2014. Nature 507: 258-261; Swarts et al., 2015. Nucleic Acids Res 43: 5120-5129) and bacteriophages (Kuzmenko et al., 2020. Nature 587 :632-637).

In contrast to eAgo proteins, the guide and target preferences of pAgo proteins are not limited to RNA: certain pAgo proteins utilize single stranded DNA (ssDNA) guides to target and cleave invading DNA sequences (Swarts et al., 2014. Nature 507: 258-261; Swarts et al., 2015. Nucleic Acids Res 43: 5120-5129; Hegge et al., 2019. Nucleic Acids Res 47: 5809-5821; Schneider et al., 2017. Nat Microbiol 2: 17035) while other pAgo proteins use RNA guides and/or act on RNA targets (Olovnikov et al., 2013. Mol Cell 51: 594-605; Doxzen et al., 2017. PLoS One 12: 1-14; Kaya et al., 2016. Proc Natl Acad Sci 113: 4057-4062; Lapinaite et al., 2018. Proc Natl Acad Sci 115: 3368-3373). Next to their mechanistic divergence, pAgo proteins are diversified in terms of domain composition (Makarova et al., 2009. Biol Direct 4: 29; Ryazansky et al., 2018. MBio 9: 1-20; Swarts et al., 2014. Nat Struct Mol Biol 21: 743-753): all eAgo proteins and most characterized pAgo proteins have a fixed four-domain composition (N-PAZ-MID-PIWI) (Lisitskaya et al., 2018. Nat Commun 9: 5165), but certain pAgo proteins contain the MID and PIWI domains only. These so called 'short pAgo proteins' form a phylogenetic clade that is separated from other pAgo proteins (Swarts et al., 2014. Nat Struct Mol Biol 21: 743-753; Makarova et al., 2009. Biol Direct 4: 29; Ryazansky et al., 2018. MBio 9: 1-20). While short pAgo proteins form the majority (57%) of all pAgo proteins (Ryazansky et al., 2018. MBio 9: 1-20), their function remained unknown.

93% of all short pAgo proteins are encoded in the same operon as (or fused to) a protein consisting of an Analog of PAZ (APAZ) domain. The APAZ domain is predicted to be homologous to the N-domain in eAgo proteins (Burroughs et al. 2014. WIREs RNA 5: 141-181). APAZ domains are commonly fused to putative nuclease domains including a Silent information regulator 2 (SIR2) domain, a Modification requiring restriction (Mrr) domain, or a Toll/Interleukin receptor (TIR) domain (Ryazansky et al., 2018. MBio 9: 1-20; Swarts et al., 2014. Nat Struct Mol Biol 21: 743-753). We coin the term 'pAgo systems' for pAgo proteins that are encoded in the same operon and are hypothesized to function in conjunction with these associated protein domains. It was recently shown that a short pAgo protein and an Mrr-APAZ protein co-purify and it was suggested that this complex mediates DNA cleavage in a guide-dependent manner (Kim et al., 2020. Biochem Biophys Res Commun 525: 755-758). Yet, the biological role of short pAgo systems and their molecular mechanisms remain completely unexplored.

### BRIEF DESCRIPTION OF THE INVENTION

Here, we describe the mechanism and application of short prokaryotic Argonaute systems. Short pAgo proteins, when combined with an effector protein comprising a TIR domain and an APAZ domain, or when combined with an effector protein comprising a SIR2 domain and an APAZ domain, form a heterodimeric Short Prokaryotic ARgonaute TIR-APAZ (SPARTA) complex or a heterodimeric Short Prokaryotic ARgonaute SIR2-APAZ (SPARSA) complex that is catalytically inactive in its apo-state. It is shown herein that, upon RNA-guided ssDNA target binding by the short pAgo, the TIR-APAZ protein is catalytically activated. This does not result in the anticipated target nucleic acid degradation, but instead results in the conversion (hydrolysis) of a nicotinamide adenine dinucleotide (NAD⁺), or an analogue thereof, to nicotinamide (NAM) and adenosine diphosphate ribose (ADPR), or analogues thereof. SPARTA and SPARSA systems can be reprogrammed to target ssDNA sequences of choice. This facilitates the development of SPARTA-based and SPARSA-based nucleic acid detection tools.

The invention therefore provides a detection system for detection of a target nucleic acid molecule, preferably a single stranded nucleic acid molecule, comprising a short pAgo protein, comprising a MID and a PIWI domain; an effector protein comprising either a Toll-Interleukin 1 receptor (TIR) domain and an Analog of PAZ (APAZ) domain, or comprising a SIR2 domain and an APAZ domain; a guide nucleic acid (gNA) that is able to hybridize to the target nucleic acid molecule; and a nicotinamide adenine dinucleotide, or an analogue thereof.

Said target nucleic acid molecule preferably is a DNA molecule, more preferably a single stranded DNA molecule.

Said gNA preferably is a ribonucleic acid molecule (gRNA). Said gNA preferably is phosphorylated at its 5'-end. Said gNA preferably comprises at least 9 nucleotides, such as 12-40 nucleotides, preferably 17-21 nucleotides.

Said nicotinamide adenine dinucleotide or analogue thereof preferably is a nicotinamide adenine dinucleotide analogue, more preferably a nicotinamide adenine dinucleotide analogue that becomes fluorescent upon conversion of the nicotinamide adenine dinucleotide, such as ε-NAD⁺ or ε-NADP⁺.

Said short pAgo proteins and TIR-APAZ effector proteins preferably are from bacteria such as, but not limited to, *Bacillales bacterium, Crenotalea thermophila, Elioreae tepidiphila, Maliponia aquimaris, Maribacter polysiphoniae, Mesorhizobium sp., Parabacteroides distasonis,* and/or *Pannonibacter phragmitetus,* preferably originate from *Crenotalea thermophila* or from *Maribacter polysiphoniae.*

Said short pAgo proteins and SIR2-APAZ effector proteins preferably are from bacteria such as, but not limited to, *Geobacter sulfurreducens, Bradyrhizobium elhanii, Xanthomonas vesicatoria,* and/or *Pseudomonas putida.*

In a further preferred detection system according to the invention, the short pAgo protein and TIR-APAZ or SIR2-APAZ effector protein is present as a single protein.

In some organisms, a pAgo protein and a TIR-APAZ effector protein are combined in a single TIR-APAZ-pAgo protein. Such TIR-APAZ-pAgo proteins preferably are from bacteria such as, but not limited to *Rhizobium leguminosarum, Neorhizobium huautlense, Rhizobium viscosum, Labrenzia* sp. EL_126, and/or *Verrucomicrobiaceae bacterium.*

In some organisms, a pAgo protein and a SIR2-APAZ effector protein are combined in a single SIR2-APAZ-pAgo protein. Such SIR2-APAZ-pAgo proteins preferably are from bacteria such as, but not limited to, *Joostella marina, Bacteroides eggerthii, Thioalkalivibrio paradoxus,* and/or *Bacteroides fragilis.*

A preferred detection system according to the invention is or comprises a thin layer chromatography or lateral flow assay.

The invention further provides a method of detecting a target nucleic acid molecule in a sample comprising nucleic acid molecules, preferably comprising single stranded nucleic acid molecules, the method comprising the steps of contacting said sample with a short pAgo protein, comprising a MID and a PIWI domain; an effector protein comprising a Toll/Interleukin1 receptor (TIR) domain and an Analog of PAZ (APAZ) domain, or comprising a SIR2 domain and an APAZ domain, or a combination of a pAgo protein and a TIR-APAZ or SIR2-APAZ effector protein; a guide nucleic acid (gNA) that is able to hybridize to the target nucleic acid molecule; and a nicotinamide adenine dinucleotide, or an analogue thereof, and detecting conversion of said nicotinamide adenine dinucleotide, or analogue thereof. In a preferred method, said sample comprising nucleic acid molecules is contacted with the detection system according to the invention.

The nucleic acid molecules in said sample preferably comprise DNA molecules, preferably single stranded DNA molecules. In a preferred method of the invention, the sample comprising nucleic acid molecules has been amplified prior to detecting the nucleic acid molecule in said sample. Said amplification preferably is performed by polymerase chain reaction (PCR) or by isothermal amplification, preferably by strand displacement amplification (SDA), nucleic acid sequence-based amplification (NASBA), or loop-mediated isothermal amplification (LAMP).

In a preferred method of the invention, the conversion of said nicotinamide adenine dinucleotide or analogue thereof is preferably performed at a temperature between 20 °C and 75 °C, preferably between 50 °C and 65 °C.

In a preferred method of the invention, the conversion of a nicotinamide adenine dinucleotide, or analogue thereof, is determined by a colorimetric method or a fluorescent method.

### FIGURE LEGENDS

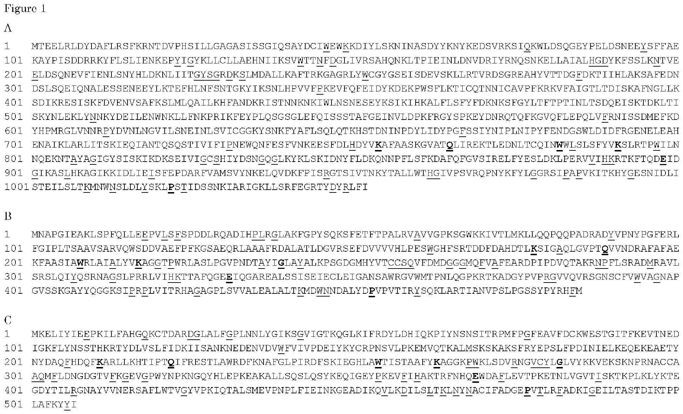

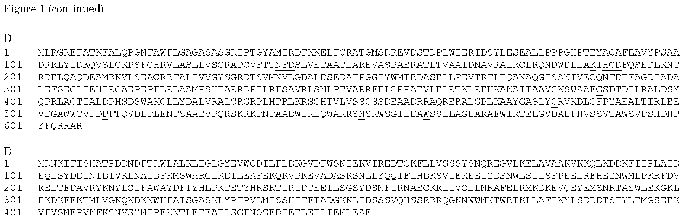

Figure 1. Exemplary amino acid sequences from pAgo proteins and effector proteins of (A) *Joostella marina* (S1A clade; SIR2-APAZ-pAgo; WP_008615787.1); (B) *Xanthomonas vesicatoria* (S1B clade; pAgo; WP_005988487.1); (C) *Maribacter polysiphoniae* (S2A clade; pAgo; WP_109649955.1); (D) *Xanthomonas vesicatoria* (S2B clade; SIR2-APAZ; WP_005988489.1); (E) *Maribacter polysiphoniae* (S2A clade; TIR-APAZ; WP_109649956.1). Bold residues are conserved in all pAgo proteins or effector proteins; underlined residues are conserved in all pAgo proteins or effector proteins from that clade.

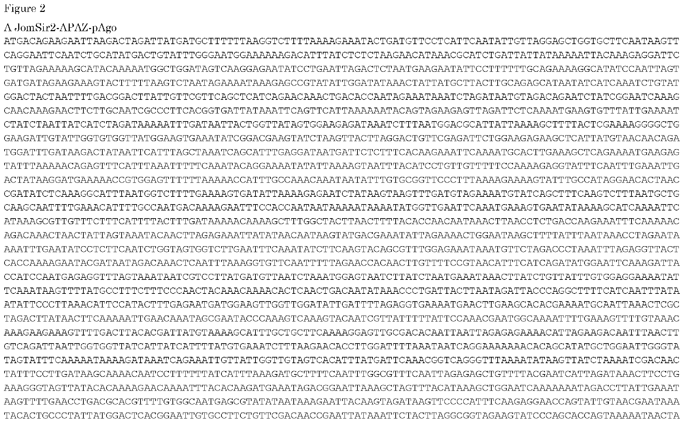

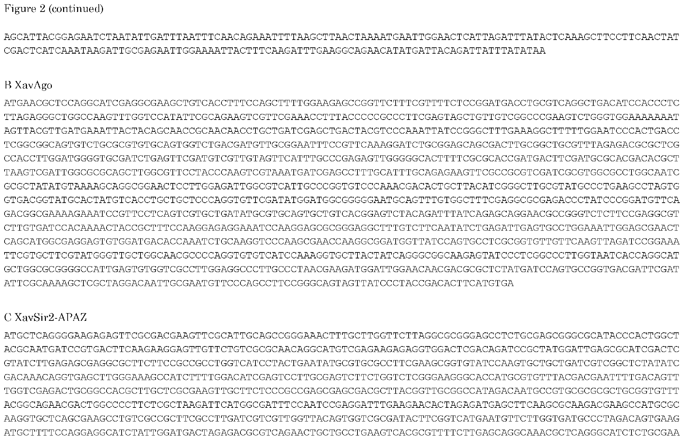

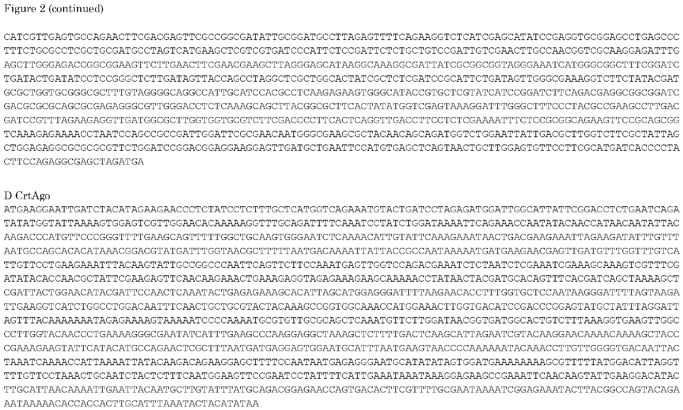

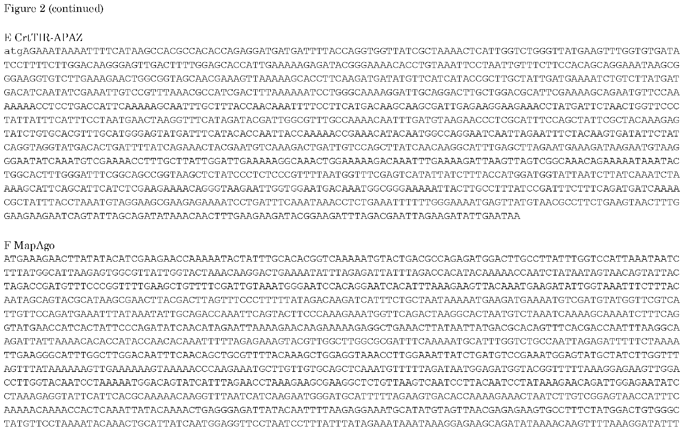

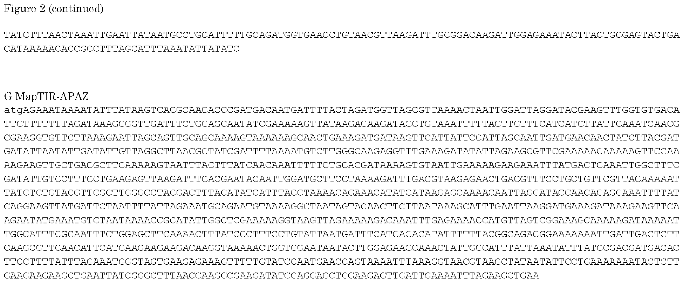

Figure 2. Sequences used. Shown are the nucleotide sequences of *Joostella marina* (Jom) SIR2-APAZ-pAgo (JomSIR2-APAZ-pAgo) (A); *Xanthomonas vesicatoria* (Xav) pAgo (XavAgo) (B) and XavSIR2-APAZ (C); *Crenotalea thermophila* (Crt) pAgo (CrtAgo) (D) and Crt TIR-APAZ (E), *Maribacter polysiphoniae* (Map) Ago (MapAgo) (F), and Map TIR-APAZ (G).

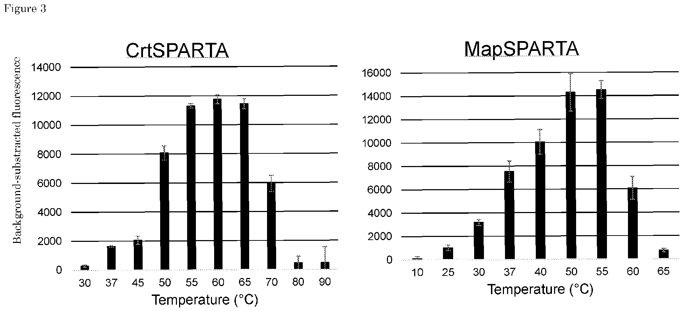

Figure 3. Temperature optimum for MapSPARTA and CrtSPARTA. Reactions with SPARTA complexes were incubated at various temperatures. After 1 h end-point fluorescence was measured and background fluorescence was subtracted. MapSPARTA (right) is active between 25 °C and 60 °C, with the optimum at 50 °C. CrtSPARTA (left) is active in the range of 37 °C and 70 °C with the optimum between 55 °C and 65 °C.

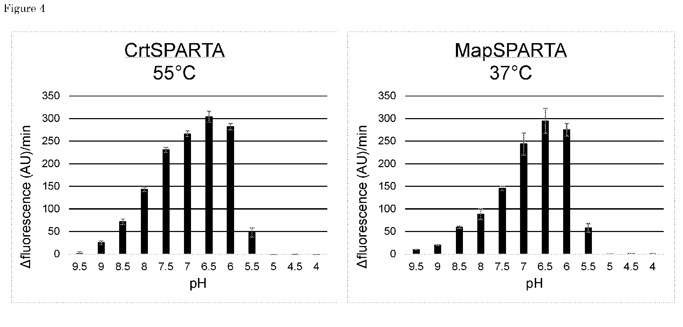

Figure 4. pH range optimum for CrtSPARTA and MapSPARTA. SPARTA systems were incubated in a reaction mix with equimolar ratio of BST, MES and citrate buffer ranging in pH from 4.0 to 9.5. Change in ε-NAD fluorescence was determined by kinetic measurements. The slope of the linear part of the curve was determined and plotted in the graph. For both MapSPARTA (right) and CrtSPARTA (left), an optimum activity was observed at a pH between 6.0 and 6.5. Their activity dropped drastically below pH 6 or above pH 7.5. Activity was observed at pH 8.5 and pH 9 and on the lower end at pH 5.5, but not at pH 5.

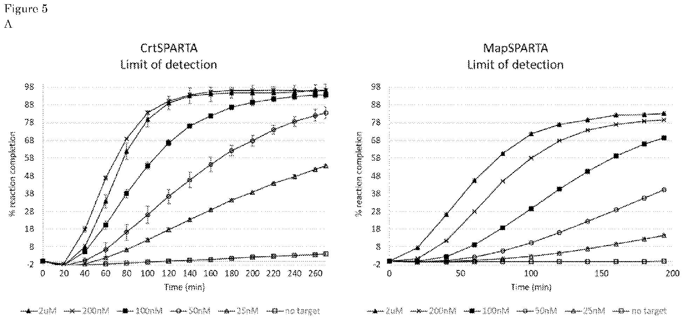

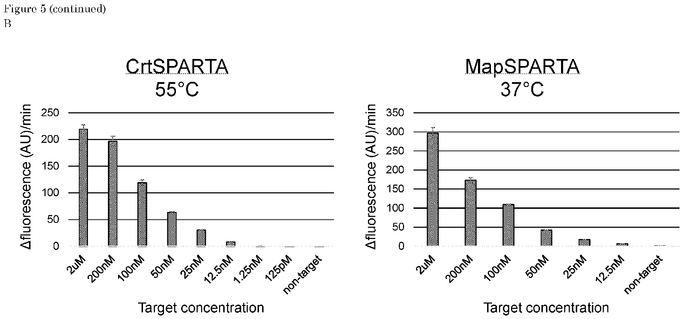

Figure 5. Limit of detection for CrtSPARTA and MapSPARTA. SPARTA systems were incubated with various concentrations of target ssDNA. Change in ε-NAD⁺ fluorescence was determined by kinetic measurements (A). The slope of the linear part of the curve was determined and plotted in the graph (B). Both CrtSPARTA (left) and MapSPARTA (right) are able to detect single stranded DNA in lower nM concentrations (25nM).

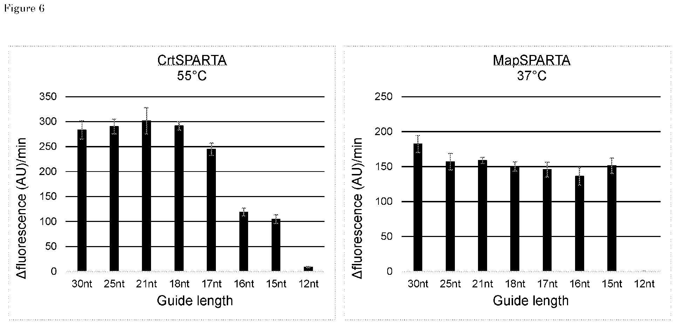

Figure 6. Effect of gRNA length on SPARTA activity. SPARTA systems were incubated with gRNAs of various lengths (12-30 nucleotides; Table 1) and a target ssDNA. Change in ε-NAD fluorescence was determined by kinetic measurements. The slope of the linear part of the curve was determined and plotted in the graph. Activity of MapSPARTA (right) gradually decreases with shorter guide lengths and is completely abolished with a 12 nucleotide-long gRNA. CrtSPARTA (left) shows little variation between guides with lengths between 18-30 nucleotides, but its activity drastically drops when 16 nucleotide-long (or shorter) guides are provided, exhibiting minimal activity with 12 nucleotide-long guide RNA.

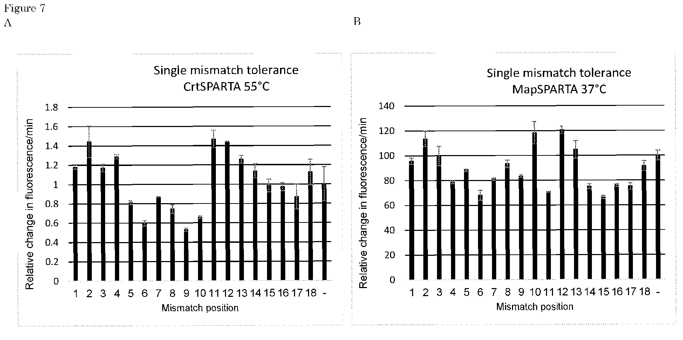

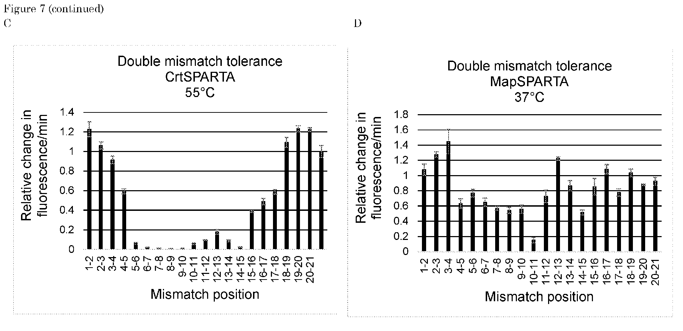

Figure 7. Mismatch sensitivity of SPARTA complexes. Mismatch sensitivity was determined by incubating CrtSPARTA complexes (A, C) and MapSPARTA complexes (B, D) with mismatched guide-target sequences (see Table 1). Change in ε-NAD⁺ fluorescence was determined by kinetic measurements. The slope of the linear part of the curve was determined and plotted in the graph.

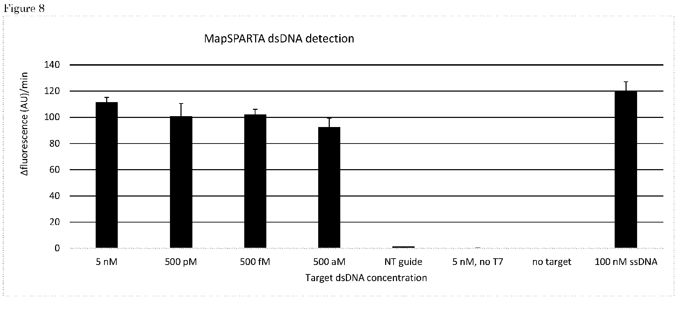

Figure 8. SPARTA combined with PCR and T7 exonuclease facilitates the detection of dsDNA at attomolar (aM) levels. MapSPARTA was mixed with guide RNA in a 1:1 molar ratio. PCR-amplified, T7 exonuclease-digested target DNA (pAP001) was added. After the addition of ε-NAD+, total fluorescence was measured over time. The slope of the linear part of the curve was determined and plotted in the graph.

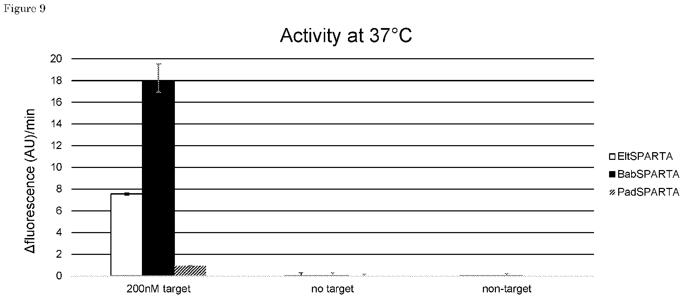

Figure 9. Various SPARTA systems were incubated with guide RNA and ssDNA target at 37°C. Change in ε-NAD+ fluorescence was determined by kinetic measurements. The slope of the linear part of the curve was determined and plotted in the graph.

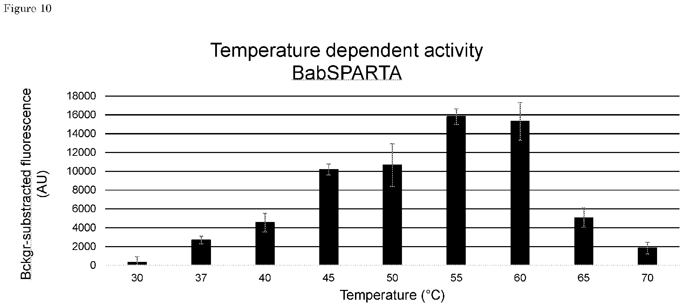

Figure 10. Temperature optimum for BapSPARTA. Reactions with SPARTA complexes were incubated at various temperatures. After 1 h end-point fluorescence was measured and background fluorescence was subtracted. BapSPARTA is active in the range of 37 °C and 70 °C with an optimum of 55 °C.

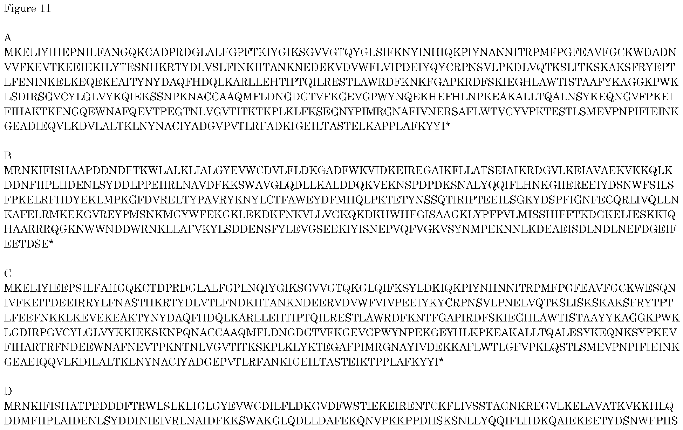

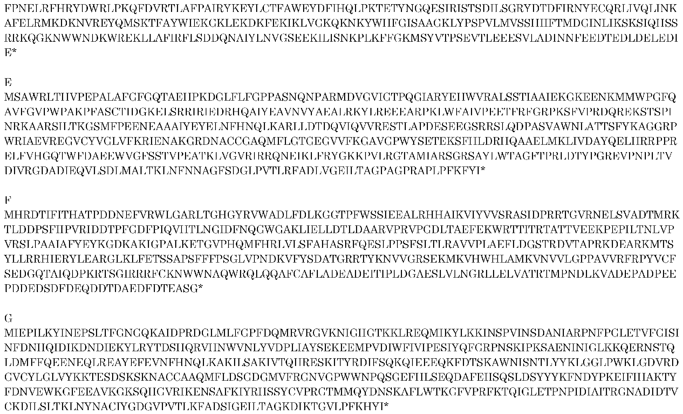

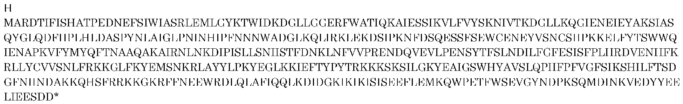

Figure 11. Amino acid sequences from pAgo proteins and TIR-APAZ effector proteins of *Bacillales bacterium* (A and B), *Crenotalea thermophila* (C and D), *Elioreae tepidiphila* (E and F), and *Parabacteroides distasonis* (G and H).

### DETAILED DESCRIPTION OF THE INVENTION

### Definitions

The term "middle (MID) domain", as used herein, refers to a domain of an Argonaute protein that binds the 5' end of a nucleic acid molecule, preferably a RNA molecule, preferably a RNA molecule with a 5' mono-phosphate. The interaction is likely to occur in a predicted Rossmann-like fold that forms a binding pocket that binds a divalent metal and contains positively charged amino acids for coordination of the phosphate group (Makarova et al., 2009. Biol Direct 4: 29; Swarts et al., 2014. Nat Struct Mol Biol 21: 743-753; Ryazansky et al., 2018. MBio 9: 1-20).

The term "P-element Induced WImpy testis in *Drosophila* (Piwi)", as used herein, refers to a part of an Argonaute protein having a RNase H endonuclease-like structure (Wei et al., 2012. J Cell Biochem 113: 2576-2585; Makarova et al., 2009. Biol Direct 4: 29; Swarts et al., 2014. Nat Struct Mol Biol 21: 743-753; Ryazansky et al., 2018. MBio 9: 1-20). A PIWI domain is not known to have NADase activity.

The term "Analog of PAZ (APAZ) domain", as used herein, refers to a domain of unknown function that is strictly found in relation to short pAgo proteins, either as a APAZ-pAgo fusion protein, or as part of a separate APAZ effector protein that is encoded in the same operon as a pAgo protein. In both cases, the APAZ domain is usually fused to another domain, for example a SIR2, TIR, or Mrr-like domain (Makarova et al., 2009. Biol Direct 4: 29; Swarts et al., 2014. Nat Struct Mol Biol 21: 743-753; Ryazansky et al., 2018. MBio 9: 1-20).

The term "Toll/Interleukin1 receptor (TIR) domain", as used herein, refers to a domain of an TIR-APAZ effector protein that is homologous to Toll/Interleukin1 receptor domains. Said TIR-APAZ effector protein is either part of a TIR-APAZ-pAgo fusion protein, or is encoded in the same operon as an Argonaute protein (Makarova et al., 2009. Biol Direct 4: 29).

The term "Silent information regulator 2 (SIR2) domain", as used herein, refers to a domain of a SIR2-APAZ effector protein, or of a SIR2-APAZ-pAgo protein, which is homologous to the SIR2 domain in Sirtuin proteins that are protein deacetylases (Makarova et al., 2009. Biol Direct 4: 29); Blander & Guarente., 2004. Annual Reviews of Biochemistry 73:417-435).
(Makarova et al., 2009. Biol Direct 4: 29).

The term "short pAgo", as used herein, refers to a prokaryotic Argonaute protein comprising a MID and PIWI domain, but lacking a PAZ domain, lacking an N-terminal (N) domain, and lacking a catalytic DEDX (where X is D, H, or K) tetrad that is typically present in certain eukaryotic Argonaute proteins and in certain long pAgo proteins and is required for cleavage of target nucleic acid molecules.

The term "TIR-APAZ effector protein" or "effector protein comprising a Toll/Interleukin1 receptor (TIR) and a Analog of PAZ (APAZ) domain", as used herein, refers to a protein comprising a TIR domain and an APAZ domain that can function as an effector when complexed with a short pAgo protein or when fused to a short pAgo protein.

The term "SIR2-APAZ protein" or "effector protein comprising a SIR2 domain and an Analog of PAZ (APAZ) domain", as used herein, refers a protein comprising a SIR2 domain and an APAZ domain that can function as an effector when complexed with a short pAgo protein or when fused to a short pAgo protein.

Short pAgo proteins and their effector proteins (e.g. TIR-APAZ proteins or SIR2-APAZ effector proteins) are described in Makarova et al., 2009. Biol Direct 4: 29, Ryazansky et al., 2018. MBio 9: 1-20; Nat Struct Mol Biol 21: 743-753.

The term "SPARTA", as used herein, which is an abbreviation of Short Prokaryotic ARgonaute TIR-APAZ, refers to a system comprising a short pAgo and a TIR-APAZ effector protein, or a fusion thereof (TIR-APAZ-pAgo).

The term "SPARSA", as used herein, which is an abbreviation of Short Prokaryotic ARgonaute SIR2-APAZ, refers to a system comprising a short pAgo and a SIR2-APAZ effector protein, or a fusion thereof (SIR2-APAZ-pAgo).

The term "operon", as is used herein, refers to a group of genes that is controlled by one and the same set of transcriptional regulatory elements such as an operator and promoter. The genes are transcribed into a single RNA strand. Genes within an operon are often functionally related.

The term "guide nucleic acid (gNA)", as is used herein, refers to single stranded nucleic molecule that comprise a sequence that guides a pAgo protein, comprising a MID and a PIWI domain, to a complementary strand of a target nucleic acid sequence. Said target nucleic acid may be single stranded or double stranded, RNA or DNA, depending on the type of system. Said gNA preferably has a length of at least 9 nucleotides, more preferred at least 17 nucleotides. Said gNA preferably is less than 1000 nucleotides, preferably less than 200 nucleotides, preferably less than 100 nucleotides. A preferred length is between 17 and 35 nucleotides, such as between 18 and 30 nucleotides. Said gNA molecule may include nucleic acid nucleotide analogues such as inosine, uridine, xanthine, hypoxanthine, 2,6-diaminopurine, and 6,8-diaminopurine-based ribonucleotides and deoxyribonucleotides. Short Argonaute protein(s) preferably in complex with or fused to TIR-APAZ or SIR2-APAZ effector proteins assembled around a gNA will form a nucleoprotein (NP) complex that specifically binds a complementary target sequence.

The term "complementary strand", as is used herein in the context of gNA, refers to a target nucleic acid strand that is substantially complementary to, i.e. can base pair with, a specific gNA. Said complementary strand will bind to, and base pair with, the gNA upon contacting the gNA and target nucleic acid under suitable conditions. Said complementary strand is either a single stranded target molecule, or one of the strands of a double stranded target molecule. In the latter case, binding of the gNA to the complementary strand will displace the non-complementary strand of the target molecule.

The term "suitable conditions", as is used herein include a temperature between 20 °C and 75 °C, preferably between 37 °C and 70 °C, preferably between 50 °C and 65 °C, a pH between 5 and 9, preferably between 6 and 7.5, ionic strength of 10-1000 millimole/L, preferably at an ionic strength of 20-500 millimole/L. Said ionic strength preferably is provided by a buffer or buffers and/or a salt or salts. Said salt preferably comprises a manganese and/or magnesium salt, preferably manganese chloride and/or magnesium chloride.

The term "NADase activity", as is used herein, refers to an enzymatic activity that converts (hydrolyses) a nicotinamide adenine dinucleotide (NAD⁺), or an analogue thereof, to nicotinamide (NAM) and adenosine diphosphate ribose (ADPR), or the corresponding analogue or analogues thereof.

The term "NAD⁺ analogue", as is used herein, refers to an analogue of NAD⁺ that can be hydrolysed by a SIR2 or TIR NADase. The term analogue includes reference to nicotinamide adenine dinucleotide phosphate (NADP⁺) and modified NAD⁺ or NAPD⁺ molecules such as radiolabeled or fluorescent molecules, including but not limited to β-nicotinamide- N⁶- (2- (6- [fluoresceinyl]aminohexanoyl) aminoethyl)adenine dinucleotide, B-nicotinamide-N6-[2-[[6-[fluorescein]-amino]-2-oxohexyl-hexyl]amino]-2-oxoethyl]adenine dinucleotide, nicotinamide 1,N⁶-ethenoadenine dinucleotide (ε-NAD⁺), and β- nicotinamide- 1, N⁶- ethenoadenine dinucleotide phosphate ( ε-NADP⁺).

The term "reverse transcribing", as is used herein, refers to the generation of complementary DNA (cDNA) from an RNA template. Retroviruses and some retrotransposons encode enzymes, termed reverse transcriptase, to replicate their genomes. A retroviral reverse transcriptase has three sequential biochemical activities: RNA-dependent DNA polymerase activity, ribonuclease H (RNase H), and DNA-dependent DNA polymerase activity. Enzymes that are often used for reverse transcribing RNA are Moloney murine leukemia virus reverse transcriptase and avian myeloblastosis virus reverse transcriptase, and variants thereof, including thermostable variants.

The term "host cell", as is used herein, includes a prokaryotic cell and a eukaryotic cell such as a yeast cell or a mammalian cell.

The term "prokaryote", as is used herein, refers to a cellular organism that lacks an envelope-enclosed nucleus. Cellular organisms with a nucleus enclosed within a nuclear envelope are referred to as eukaryotes. Prokaryotes include true bacteria (eubacteria), and archeae (archaeabacteria).

The term "eukaryotic cell", as is used herein, refers to cell that has a nucleus enclosed within a nuclear envelope, such as a protist, fungal, plant, or animal, including human, cell.

The term "expression vector", as is used herein, refers to a vector that is able to direct expression of one or more genes to which they are operatively-linked. Said vector is either a plasmid, including a cosmid and a phagemid, a bacteriophage-based vector, or a viral vector. Suitable regulatory elements include promoters, enhancers, internal ribosomal entry sites (IRES), and other expression control elements such as 5' untranslated regions, optionally containing a ribosome binding site, 3' untranslated region optionally comprising a 'post stop-codon, ante terminator' region, terminator sequences, and transcription termination signals such as polyadenylation signals and poly-U sequences. For more information the average skilled person is referred to, for example, Goeddel, (1990), Gene Expression Technology in Methods in Enzymology vol 185, Academic Press. Regulatory elements include those giving direct constitutive expression in many types of host cell and those that direct expression of the nucleotide sequence only in certain cells (i.e., tissue-specific regulatory sequences). Regulatory elements may also direct expression in a temporal-dependent manner, such as in a cell-cycle dependent or developmental stage-dependent manner, which may or may not also be tissue or cell-type specific. Examples of promoters include pol I, pol II, pol III (e.g. U6 and H1 promoters). Examples of pol II promoters include, but are not limited to, retroviral Rous sarcoma virus (RSV) LTR promoter (optionally with the RSV enhancer), the cytomegalovirus (CMV) promoter (optionally with the CMV enhancer), the SV40 promoter, the dihydrofolate reductase promoter, the beta-actin promoter, the phosphoglycerol kinase (PGK) promoter, and the EF1a promoter. As well as promoters, regulatory elements may include enhancer elements, such as the woodchuck hepatitis post-transcriptional regulatory element (WPRE); CMV enhancers; the R-U5' segment in LTR of HTLV-I; SV40 enhancer; and the intron sequence between exons 2 and 3 of rabbit beta-globin. It will be appreciated by those skilled in the art that the design of the expression vector can depend on such factors as the choice of the host cell to be transformed, the level of expression desired, etc. Said regulatory elements such as promoter sequences may be an autologous sequences, or heterologous sequences, i.e. derived from a different species.

The term "tissue-specific promoter", as is used herein, is a promoter that directs expression primarily in a desired tissue of interest, such as blood, specific organs (e.g., liver, pancreas), or particular cell types.

The term "plasmid", as is used herein, refers to a circular double stranded DNA molecule into which additional DNA segments can be inserted, such as by using standard molecular cloning techniques. Plasmids often comprise an origin of replication and a marker gene that allows to identify a cell comprising the plasmid, termed selection marker. Suitable plasmids include p15A, CoIE1, pBR322, RSF1030, CIoDF13 and derivatives thereof. Further suitable plasmids are provided, for example, under the headers "Protein purification" and "Controlled expression" at addgene.org/collections/bacterial-expression/. Said selection marker includes an antibiotic resistance gene against penicillin or derivatives thereof, aminoglycoside, chloramphenicol, macrolides such as erythromycin, azithromycin and clarithromycin, streptothricin, tetracycline, quinolone, rifampin, sulfonamide, trimethoprim, kanamycin, phleomycin D1 such as zeocin, and streptomycin. Another selection marker that is often used for positive selection is a positive selection marker that enables growth of a cell on a certain medium, for example by cloning hisB⁺ in a histidine auxotrophic cell and growing the cell in a medium lacking histidine.

The term "viral vector", as is used herein, includes reference to viral nucleic acid sequences that can be packaged into a viral particle. Examples of viral vectors include retroviruses, replication defective retroviruses, adenoviruses, replication defective adenoviruses, and adeno-associated viruses. Packaging is often mediated by a packaging signal on the viral nucleic acid molecule, and performed in an appropriate packaging cell that expresses the required proteins.

The terms "base pairing affinity" and "complementarity", as are used herein, refer to the ability of a nucleic acid to form hydrogen bond(s) with another nucleic acid sequence by either traditional Watson-Crick base pairing or other non-traditional types. A percent identity (i.e. complementarity) in relation to a reference sequence, in the various descriptions of the invention, represents the percentage of residues in a nucleic acid molecule which can form hydrogen bonds (e.g., Watson-Crick base pairing) with a second nucleic acid sequence, preferably over the complete length of the shortest sequence (e.g., 5, 6, 7, 8, 9, 10 out of 10 being 50%, 60%, 70%, 80%, 90%, and 100% identity). A person of skill in the art will appreciate that the actual base pairing may further depend on, for example, the actual sequences of the target nucleic acid and gNA, type (for example G-U or G-A mismatch) and position of the mismatch, the reaction conditions including temperature and ionic strength, and on the specific short pAgo system, e.g. SPARTA/SPARSA system, that is being used.

The term "perfectly complementary", as used herein, indicates that all contiguous residues of a nucleic acid sequence in a gNA will hydrogen bond with the same number of contiguous residues in a target nucleic acid sequence, corresponding to a 100% identity over the whole length of the complementary sequences.

The term "substantially complementary", as is used herein, refers to a degree of identity that is at least 90%, 95%, 97%, 98%, 99%, or 100% between the portion of a gNA and the equivalent length of a targeting nucleic acid molecule. Mismatches between gNA and a target nucleic acid molecule are allowed as long as they permitgNA-target nucleic acid base pairing and subsequent pAgo-mediated activation of the SIR2-APAZ or TIR-APAZ NADase activity. A limited number of mismatches, such as one, two or three mismatches between gNA and target nucleic acid molecule are allowed. Said one two or three mismatches preferably are not at nucleotide positions 6, 7, or 9 from the 5'-end of the complementary of the gNA, more preferably not between nucleotide positions 6-10 from the 5'-end of the complementary of the gNA.

The term "stringent conditions", as used herein, in the context of hybridization conditions, refer to conditions under which a nucleic acid having complementarity to a target sequence predominantly hybridizes with the target sequence, and substantially does not hybridize to non-target sequences. Stringent conditions are generally sequence-dependent and vary depending on a number of factors. In general, the longer the sequence, the higher the temperature at which the sequence specifically hybridizes to its target sequence. Calculations regarding hybridization conditions required for attaining particular degrees of stringency are discussed in Sambrook et al., Molecular Cloning: A Laboratory Manual (Cold Spring Harbor Laboratory Press, Cold Spring Harbor, NY, 2001); and Tijssen, Laboratory Techniques in Biochemistry and Molecular Biology - Hybridization with Nucleic Acid Probes Part I, Chapter 2 (Elsevier, New York, 1993). The Tm is the temperature at which more than 50% of a given strand of a nucleic acid molecule is hybridized to its complementary strand.

### Short Argonaute pAgo and effector proteins

Previous bioinformatics studies have identified various short pAgo protein-encoding genes (Makarova et al., 2009. Biol Direct 4: 29; Swarts et al., 2014. Nat Struct Mol Biol 21: 743-753; Ryazansky et al., 2018. MBio 9: 1-20). These studies were performed on the complete RefSeq database, including unassembled genomic sequences, which might hamper identification of proteins genetically associated with short pAgo proteins. Therefore, we conducted a hidden Markov Model (HMM)-based homology search for pAgo proteins using the NCBI RefSeq database with 7249 proteomes that correspond to only representative, reference, chromosome and scaffold level assemblies. This analysis resulted in the identification of 283 short pAgo proteins that are comprised of the MID and PIWI domains, and it was confirmed that they all lack a catalytic DEDX tetrad (Makarova et al., 2009. Biol Direct 4: 29; Swarts et al., 2014. Nat Struct Mol Biol 21: 743-753; Ryazansky et al., 2018. MBio 9: 1-20) required by long pAgo proteins for target cleavage (Swarts et al., 2015. Nucleic Acids Res 43: 5120-5129; Hegge et al., 2019. Nucleic Acids Res 47: 5809-5821). Maximum-likelihood based phylogenetic analysis of these short pAgo proteins revealed four subclades, which were named S1A, S1B, S2A, and S2B (data not shown).

To identify genes that specifically associate with short pAgo encoding-genes, we analysed their genomic neighborhoods. In clade S1A, all short pAgo proteins are fused to SIR2 and APAZ domains. In contrast, no short pAgo-fusions were observed in other clades. However, a putative TIR-APAZ-pAgo fusion protein-encoding gene has been identified by Ryazansky et al., 2018. MBio 9: 1-20. Instead, short pAgo proteins in clades S1B, S2A, and S2B are encoded in an operon together with an APAZ domain-containing protein. Short pAgo genes from clade S1B strictly associate with SIR2-APAZ-encoding genes, while short pAgo genes from clade S2A strictly associate with TIR-APAZ-encoding genes. Short pAgo genes from clade S2B are mainly associated with DUF4365-APAZ proteins (homologous to the RecB-like domain of Mrr proteins (Ryazansky et al., 2018. MBio 9: 1-20) and to a limited extent with proteins that consist of an APAZ domain fused to a DHS-like NAD/FAD-binding superfamily domain. To investigate if short pAgo proteins might function in conjunction with additional proteins, we further analysed genes in their genomic neighborhood. No genes other than the APAZ-containing proteins were specifically associating with short pAgo-encoding genes. This implies that these systems either function as stand-alone systems or that they rely on common host factors for their activity. As short pAgo systems strictly associate with (or are fused to) APAZ-domain containing proteins, but not with other proteins, we coin the term 'short pAgo system' to indicate a short pAgo and its associated APAZ domain-containing protein (clades S1B, S2A, and S2B), or a fusion thereof (clade S1A).

Short pAgo systems in the S1A clade and S1B clades are termed 'SPARSA-A and SPARSA-B systems', respectively, for short prokaryotic Argonaute SIR2-APAZ systems. Without exception, all SPARSA system-APAZ domains are fused to a SIR2 domain.

Short pAgo systems in the S2A clade are named 'SPARTA systems' for short prokaryotic Argonaute TIR-APAZ systems. Without exception, all SPARTA system-APAZ domains are fused to a TIR domain. In one subclade of SPARTA systems, a putative modification requiring restriction endonuclease (Mrr) domain is fused to the TIR-APAZ protein. Given the homology of pAgo-associated SIR2 and TIR domains to known nucleases, it has been hypothesized that these domains could compensate the catalytic activity of the PIWI domain that is lost in short pAgo proteins (Hegge et al., 2017. Nature Reviews Microbiology 16:5-11). As such, short pAgo proteins could facilitate gNA-mediated target nucleic acid molecule recognition, which would result in target degradation by the APAZ-associated domain. However, no catalytic activity has until now been demonstrated for short pAgo-associated SIR2 and TIR domains. SIR2 domains are found in Sir2 (Sirtuin) family proteins which are generally known as NAD-dependent protein deacetylases (Buck et al., 2004. J Leukoc Biol 75: 939-950). On the contrary, TIR domains have been considered as scaffolding domains that mediate protein-protein interactions without any catalytic activity (Luo et al., 2020. J Cell Sci 133: jcs239194). However, in bacteria, some TIR and SIR2 domains which are not associated with short pAgo proteins have been reported to show NADase activity (Essuman et al., 2018. Current Biol 28: 421-430; Ka et al., 2020. Nat Commun 2816: 1-8).

Examples of pAgo proteins and associated effector proteins are provided in Figure 1. Amino acid residues that are conserved in pAgo proteins or effector proteins within one clade are underlined, while amino acid residues that are conserved in all pAgo proteins and/or effector proteins are provided in bold and underlined. Additional pAgo proteins and associated effector proteins are provided in Figure 11. Sequence identity matrices of these pAgo proteins are provided in Table 2 (A and B).

In general, S1A clade SIR2-APAZ-pAgo proteins are between 1002 and 1060 amino acids in length and consist of a SIR2 domain-APAZ domain-MID domain-PIWI domain. When based on a *Joostella marina* pAgo protein of 1052 amino acids long (WP_0008615787.1), residues 1-159 constitute a SIR2 domain, residues 160-581 from an APAZ domain, residues 582-816 form a MID domain, and residues 817-1052 form a PIWI domain.

Conserved SIR2 domain residues in all S1A pAgo proteins include W46, K49, Q77, Y95, R109, P122, G125, W142, N145, and D147. One conserved motif Y-X₁₃-R-X₁₂-P-X₂-G-X₁₆-W-X₂-N-X-D is present in all S1A SIR2 domains. Semi conserved residues in S1A SIR2 domains include amino acid positions 40 (S or T), 41 (A or G), 44 (C or L), 48 (W or F), 52 (I or L), 76 (I or L), 79 (W or Y), 94 (E or P), 98 (Y or F), 112 (Y or F), 132 (L or M), 143 (T or S), 144 (T or L), and 146 (F or L).

Conserved APAZ domain residues in all S1A pAgo proteins include H187, G188, D189, N197, E201, G226, Y227, S228, G229, D231, S233, W252, F285, D286, N338, P345, P482 and N510. Two conserved motifs H-G-D-X₇-N-X₃-E and G-Y-S-G-X-D-X-S are present in all S1A APAZ domains. Semi conserved residues in S1A APAZ domains include amino acid positions 191 (K or R), 192 (Y or F), 196 (K or Q), 198 (T or S), 202 (L or I), 224 (V or I), 230 (R or S), 235 (M or L), 250 (L or I), 266 (L or A), 284 (G or S), 324 (F or I), 520 (W or F), 544 (F or L) and 579 (G or S).

Conserved MID domain residues in all S1A pAgo proteins include F589, P612, F667, P678, P731, H750, K754, Q765, W782, L784, K791, W797, A807 and G810. Two conserved motifs P-X₁₈-H-X₃-K-X₁₀-Q and W-X-L-X₆-K-X₅-W-X₉-A-X₂-G are present in all S1A MID domains. Semi conserved residues in S1A MID domains include amino acid positions 584 (E or D), 602 (H or N), 603 (P or Q), 606 (G or A), 607 (L or V), 614 (D or E), 633 (P or G), 660 (Y or F), 664 (Y or F), 666 (G or S), 671 (Y or F), 675 (L or I), 729 (F or Y), 748 (D or N), 751 (D or N), 755 (A or L), 771 (T or S), 785 (S or A), 786 (L or T), 789 (Y or F), 790 (V or A), 796 (P or L), 808 (Y or F), 811 (I or L), 812 (G or S), 813 (Y or F), and 814 (S or A).

Conserved PIWI domain residues in all S1ApAgo proteins include G827, S829, G836, G838,V887, H889, K890, E898, G901, L906, I919, R946, H962, G963, G978, P983, P985, G993, K1009, N1013, Y1018, P1022, T1024, D1047 and R1049. Three conserved motifs G-X-S-X₆-G-X-G; V-X-H-K-X₇-E-X₂-G-X₄-L, and G-X₄-P-X-P-X₇-G-X₁₅-K-X₃-N are present in all S1A PIWI domains. Semi conserved residues in PIWI domains include amino acid positions 828 (C or S), 830 (H or Q), 832 (Y or F), 843 (L or M), 857 (P or S), 859 (L or M), 863 (D or E), 864 (A or S), 885 (R or K), 892 (T or G), 897 (D or E), 915 (D or E), 916 (L or M), 926 (R or K), 942 (F or Y), 944 (I or V), 947 (G or A), 958 (L or Y), 975 (Y or F), 982 (I or V), 990 (K or R), 1004 (I or V), 1005 (L or I), 1007 (L or F), 1008 (T or S), 1010 (M or V), 1011 (N or D), 1012 (W or F), 1028 (S or A), 1032 (A or S), 1035 (G or S), 1045 (Y or F), 1048 (Y or F), 1051 (F or L), and 1052 (I or M).

In general, S1B pAgo proteins are between 465-485 amino acids in length and consist of a MID domain fused to a PIWI domain. When based a *Xanthomonas vesicatoria* pAgo protein (WP_005988487.1) of 485 amino acids long, residues 1-247 form a MID domain, while residues 248-485 constitute a PIWI domain.

Conserved MID domain residues in all S1B pAgo proteins include E18, L21, F23, P35, L36, G38, A60, Y90, W162, K178, A182, Q189, W208, A213, K217, G220, P222, A236, G239, and Y242.

Conserved PIWI domain residues in all S1B pAgo proteins include C256, C257, S258, Q259, G265, G267, F270, A272, N288, P289, M296, Y307, G313, H322, K323, E331, R380, G381, W394, G397, P416, P418, G426, K442, W445, N446, P455, Y460, and F485. Three conserved motifs C-C-S-Q-X₅-G-X-G-X₂-F-X-A, N-P-X₆-M-X₁₀-Y-X₅-G-X₈-H-K-X₇-E and P-X-P-X₇-G-X₁₅-K-X₂-W-N-X₈-P-X₄-Y are present in PIWI domains of S1B pAgo proteins.

In general, S1B SIR2-APAZ proteins are between 534 and 623 amino acids in length and consist of a SIR2 domain fused to an APAZ domain. When based a *Xanthomonas vesicatoria* SIR2-APAZ effector protein of 607 amino acids long, residues 1-154 constitute a SIR2 domain and residues 155-607 form an APAZ domain.

Conserved SIR2 domain residues in all S1B effector proteins include Y88, F92, N138, F139, and D140, while amino acid positions 47 (Y or F), 80 (P or A), 103 (I or L), 105 (Y or F), 106 (I or L), 136 (T or S), 137 (T or P), and 144 (E or A) are semi-conserved.

Conserved APAZ residues in all S1B effector proteins include K187, H189, G190, D191, L204, G228, S230, G231, R232, D233, G253, W256, A275, G388, G481, P511, N551, and W561. Two conserved motifs K-X-H-G-D-X₁₂-L and G-X-S-G-R-D-X₁₉-G-X₂-W-X₁₈-A are present in all S1B APAZ domains. Semi conserved residues in S1B APAZ domains include amino acid positions 183 (P or A), 192 (F or Y), 199 (N or I), 200 (T or L), 208 (D or N), 223 (G or A), 228, 229 (Y or S), 235 (S or N), 236 (V or I), 268 (V or A), 334 (P or Q), 339 (N or S), 347 (P or S), 581 (F or I), and 590 (T or S).

In general, S2A pAgo proteins are between 453 and 521 amino acids in length and consist of a MID domain that is fused to a PIWI domain. When based on a *Maribacter polysiphoniae* pAgo protein (WP_109649955) or *Crenatolea thermophila* pAgo protein (WP_092459742.1) of 507 amino acids, residues 1-287 constitute a MID domain and residues 288-507 form an PIWI domain.

Conserved MID residues in all S2A pAgo proteins include E9, Q18, D25, G26, G31, G42, G77, W139, F206, K211*, Q222*, W254*, K263*, P268, W269, R275, V278, C279, Y280, G282*, and K286, whereby the asterisk indicates residues conserved in all short pAgo proteins. One conserved motif P-W-X₅-R-X₂-V-C-Y-X-G-X₃-K is present in all S2A MID domains. Semi conserved residues in MID domains of S2A pAgo proteins include amino acid positions 44 (I or V), 45 (G or A), 121 (F or Y), 151 (G or C), 152 (R or K), 183 (F or L), 207 (H or R), 215 (L or I), 253 (A or S), 261 (Y or F), 262 (Y or F), and 285 (Y or F).

Conserved MID residues in all pAgo proteins include K211, Q222, W254, K263, and G282. Semi conserved residues in MID domains of all pAgo proteins include amino acid positions G77 (G or S), P268 (P or L), W269 (W or F), and Y285 (F or Y).

Conserved PIWI residues in all S2A pAgo proteins include A301, Q302, F304, F313, G315, G318, P354, F358, H360, E369*, F373, I388, R407, G424, V455, D458, L462, K464, N466, N468, P477*, T479, F482, G487, and Y506. Three conserved motifs V-X₂-D-X₃-L-X-K-X-N-X-N-X₈-P-X-T-X₂-F-X₄-G, A-Q-X-F-X₈-F-X-G-X₂-G and P-X₃-F-X-H-X₈-E-X₃-F are present in all S2A PIWI domains. Semi conserved residues in PIWI domains of S2A pAgo proteins include amino acid positions 298 (C or V), 299 (C or S), 300 (A or G), 303 (M or L), 305 (L or I), 308 (G or S), 310 (G or A), 312 (V or I), 314 (R or K), 320 (W or F), 329 (H or K), 330 (L or I), 335 (A or S), 346 (Y or F), 356 (E or Q), 359 (I or V), 365 (F or I), 370 (W or I), 371 (G or A), 386 (V or I), 395 (K or R), 408 (G or N), 418 (A or G), 420 (L or V), 427 (P or A), 438 (P or A), 440 (P or N), 443 (V or I), 463 (T or S), 467 (Y or F), 474 (D or S), 478 (V or I), 483 (A or S), 486 (V or I), 489 (I or V), 490 (L or I), and 503 (F or L).

Conserved PIWI residues in all pAgo proteins include E369, and P477. Semi conserved residues in PIWI domains of all pAgo proteins include amino acid positions Q302 (Q or H), A372 (G or A), G424 (G or S), P438 (P or A), K464 (K or R), N466 (N or D), N468 (N or S), A483 (A or S)

In general, S2A effector TIR-APAZ proteins are between 424-513 amino acids in length, with one outlier having 624 amino acids. Some S2A effector proteins have an additional Modification requiring restriction (Mrr)-like domain at the N-terminus and comprise 639-649 amino acids in length. When based on a *Maribacter polysiphoniae* TIR-APAZ protein (WP_109649956) of 452 amino acids long, or on a *Crenatolea thermophila* TIR-APAZ protein (WP_092459739) of 450AA long, amino acids, residues 1-107 form a TIR domain, while residues 108-450 (452) constitute an APAZ domain.

Conserved TIR residues in all S2A effector proteins include W20, L25, G29 and G42, forming a conserved motif W-X₄-L-X₃-G-X₁₂-G that is present in all S2A TIR domains. Semi conserved residues in TIR domains of S2A effector proteins include amino acid positions 6 (F or L), 7 (I or L), 12 (P or L), 30 (Y or F), 32 (V or T), 35 (D or E), together forming a conserved motif P/L-X₇-W-X₄-L-X₃-G-Y/F-X-V/T-X₂-D/E-X₆-G in TIR domains of S2A effector proteins.

Conserved APAZ residues in all S2A effector proteins include W319, R361, N370, and W373, forming a conserved motif R-X₈-N-X₂-W that is present in all S2A APAZ domains. Semi conserved residues in APAZ domains of S2A effector proteins include amino acid positions 124 (W or H), 182 (P or L), 217 (F or L), and 312 (G or N).

Based on the information provided herein above and in Figures 1, 2 and 11, a person skilled in the art will be able to determine metes and bounds of short pAgo proteins, comprising a MID and a PIWI domain; and of effector proteins comprising TIR-APAZ domains or comprising SIR2-APAZ domains. Said skilled person will be able to determine whether an unknown protein is a pAgo protein, comprising a MID and a PIWI domain; or is an effector protein comprising TIR-APAZ domains or comprising SIR2-APAZ domains, or not. In addition, said person will be able to assemble chimeric proteins, such as chimeric pAgo proteins, comprising MID and PIWI domains from different organisms, and/or chimeric effector proteins comprising TIR-APAZ domains or SIR2-APAZ domains from different organisms.

Further, a short pAgo protein and a TIR-APAZ effector protein can be provided or assembled into a single polypeptide comprising a TIR domain, an APAZ domain, a MID domain, and a PIWI domain, wherein each domain originates from the same or a different species. Likewise, a short pAgo protein and a SIR2-APAZ effector protein may also be provided or assembled into a single polypeptide comprising a SIR2 domain, an APAZ domain, a MID domain, and a PIWI domain, wherein each domain originates from the same or a different species. For example, such protein fusions are found in the S1A SIR2-APAZ-pAgo clade, but also TIR-APAZ-pAgo fusions have been identified. For example, in *Rhizobium leguminosarum* (WP_081295619.1), *Neorhizobium huautlense* (WP_105374321.1), *Rhizobium viscosum* (WP_192730401.1), *Labrenzia* sp. EL_126 (WP_196916567.1), and *Verrucomicrobiaceae bacterium* (RYD86018.1), short pAgo and TIR-APAZ are present as a single polypeptide.

When generating fusion proteins, a linker may be positioned in between the individual domains. A preferred linker comprises from 1 to about 20 amino acid residues and provides flexibility to the resulting chimeric protein. Some preferred examples of such amino acid sequences include Gly-Ser linkers, for example of the type (Glyₓ Ser_{y})_{z} such as, for example, described in WO 99/42077, WO 06/040153 and WO 06/122825. A most preferred linker is a (Gly4 Ser)3 linker.

### Detection system

In an embodiment, the invention provides a detection system for detecting and/or quantifying a target nucleic acid molecule, preferably a single stranded nucleic acid molecule, said detection system comprising a short pAgo protein, comprising a MID and a PIWI domain; an effector protein comprising Toll/Interleukin1 receptor (TIR)-Analog of PAZ (APAZ) domains, or comprising SIR2-APAZ domains; or a fusion of said short pAgo protein and one of said effector proteins, a guide nucleic acid (gNA) that is able to hybridize to the target nucleic acid molecule; and nicotinamide adenine dinucleotide (NAD⁺), or an analogue thereof. Said detection system optionally comprises methods and means for analysing conversion of nicotinamide adenine dinucleotide.

Said detection system preferably comprises a purified, or at least partially purified short pAgo protein comprising a MID and a PIWI domain, and an effector protein comprising TIR-APAZ domains or SIR2-APAZ domains, or fusions thereof. Said proteins preferably originate from *Bacillales bacterium, Crenotalea thermophila, Elioreae tepidiphila, Maliponia aquimaris, Maribacter polysiphoniae, Mesorhizobium sp., Parabacteroides distasonis,* and/or *Pannonibacter phragmitetus, Geobacter sulfurreducens, Bradyrhizobium elhanii, Xanthomonas vesicatoria,* and/or *Pseudomonas putida.* Said short pAgo and effector protein can be from the same or from different species, preferably from the same species. Said Sir2-APAZ-pAgo fusion proteins preferably originate from *Joostella marina, Bacteroides eggerthii, Thioalkalivibrio paradoxus,* and/or *Bacteroides fragilis.* Said TIR-APAZ-pAgo fusion proteins preferably originate from *Rhizobium leguminosarum, Neorhizobium huautlense, Rhizobium viscosum, Labrenzia* sp. EL_126, and/or *Verrucomicrobiaceae bacterium.*

Said short pAgo protein comprising a MID and a PIWI domain may be a chimeric protein, comprising a MID domain from a first organism, and a PIWI domain from a second organism. Similarly, said effector protein comprising TIR-APAZ domains or SIR2-APAZ domains may be a chimeric protein, comprising an APAZ domain from a first organism, and a TIR or SIR2 domain from a second organism.

Said short pAgo protein and SIR2-APAZ or TIR-APAZ effector proteins may be a 'fusion' protein in which both polypeptide sequences are fused, potentially through a linker peptide.

Said detection system preferably comprises said short pAgo protein comprising a MID and a PIWI domain, and said effector protein comprising TIR-APAZ domains or SIR2-APAZ domains, in a molar ratio of between 5:1 and 1:5, preferably between 3:1 and 1:3, preferably between 2:1 and 1:2, preferably about 1:1.

Said short SPARTA or SPARSA system preferably is allowed to form a gNA-protein complex. Said gNA-pAgo-effector protein complex remains inactive in the absence of a target nucleic acid molecule. Activation is triggered by binding of the gNA to a target nucleic acid molecule that is substantially complementary to the gNA allowing base pairing of the gNA with the single stranded target nucleic acid molecule, or with one strand of a double stranded target nucleic acid molecule. It is assumed that activation of the gNA-pAgo protein complex by base-pairing of the gNA with a target nucleic acid molecule induces a conformational change in the effector protein such that the NADase activity of the effector protein becomes functional.

Said target nucleic acid molecule preferably is a deoxyribonucleic acid (DNA) molecule, which may either be single stranded or double stranded, preferably single stranded. Said target DNA molecule may be generated by reverse transcription of a RNA molecule, as is known to a person skilled in the art. Methods for reverse transcribing RNA into copy DNA (cDNA) are known in the art and include the use of a RNA-dependent DNA polymerase and universal primers, one or more specific primers, or a combination thereof.

Methods to generate a single stranded target nucleic acid molecule, such as a single stranded DNA molecule, are known in the art and include conventional melting and/or alkali treatment of a double stranded DNA molecule, asymmetric amplification, rolling circle amplification, enzymatic processing such as helicase-mediated unwinding of the DNA, selective enzymatic digestion of a 5'-phosphorylated strand from a double stranded nucleic acid product, and immobilization of a double stranded DNA molecule, for example an amplification product, of which one of the strands is labeled, for example biotinylated, followed by separation of the labeled and non-labeled strands.

As an example, a single stranded target nucleic acid molecule may be generated by amplification of a target nucleic acid molecule, followed by digestion of one of the DNA strands of the amplified nucleic acid molecule. Amplification may be performed, for example, by polymerase chain reaction (PCR), in the presence of a forward and a reverse primer, one of which comprises at least one 5'-phosphorothioate group, preferably two or more, such as three, four or five phosphorothioate groups. Amplification may be followed by incubation with a single stranded exonuclease such as T7 exonuclease (Nikiforov et al., 1994. Genome Res 3: 285-291), resulting in a single stranded target nucleic acid molecule. As an alternative, amplification may be performed in the presence of a single stranded exonuclease such as T7 exonuclease, provided that the primers are protected against degradation by the a single stranded exonuclease,

Said gNA preferably is a ribonucleic acid molecule (gRNA). Said gNA molecule may include ribonucleic acid nucleotide analogues such as inosine, uridine, xanthine, hypoxanthine, 2,6-diaminopurine, and 6,8-diaminopurine-based ribonucleotides and deoxyribonucleotides.

Said gNA, preferably gRNA, is preferably phosphorylated at its 5'-end. Said gNA, preferably gRNA, is preferably generated, for example, by chemical synthesis using, for example, the phosphoramidite method of Marvin H. Caruthers (Caruthers, 1985. Science 230: 281-285). Said chemical synthesis may employ phosphoramidites such as TheraPure phosphoramidites (Thermo Fisher Scientific, Waltham, Massachusetts). Said 5' phosphate group may be added during synthesis (Guzaev et al., 1995. Tetrahedron 51: 9375-9384), or added after synthesis, for example by the transfer of a gamma-phosphate of ATP to the 5'-hydroxyl termini of said gNA, preferably gRNA, for example by a polynucleotide kinase such as a T4 polynucleotide kinase.

Said gNA, preferably gRNA, preferably comprises at least 9 nucleotides, more preferably at least 12 nucleotides, such as 12-40 nucleotides, preferably 14-35 nucleotides, preferably 15-30 nucleotides, preferably 15-25 nucleotides, preferably 16-23 nucleotides, preferably 17-21 nucleotides, including 18 nucleotides, 19 nucleotides and 20 nucleotides.

For the *in vitro* assembly of a gNA-protein complex, the required pAgo proteins and effector proteins may be expressed and purified from a suitable expression system. Commonly used expression systems for heterologous protein production include *E*. *coli, Bacillus* spp., baculovirus, fungi including filamentous fungi and yeasts such as *Saccharomyces cerevisiae* and *Pichia pastoris,* eukaryotic cells such as Chinese Hamster Ovary cells (CHO), human embryonic kidney (HEK) cells and PER.C6^{®} cells (Thermo Fisher Scientific, MA, USA), and plants or plant cells. The efficiency of expression of recombinant proteins in heterologous systems depends on many factors, both on the transcriptional level and the translational level.

Short pAgo and effector proteins preferably are produced using prokaryotic cells such as *E. coli.* Said pAgo and effector proteins are preferably produced by expression cloning of the proteins in a prokaryotic cell of interest, preferably *E*. *coli.* For this, an expression construct, preferably DNA, is preferably produced by recombinant technologies, including the use of polymerases, restriction enzymes, and ligases, as is known to a skilled person. Alternatively, said expression construct is provided by artificial gene synthesis, for example by synthesis of partially or completely overlapping oligonucleotides, or by a combination of organic chemistry and recombinant technologies, as is known to the skilled person.

As an alternative, or in addition, pAgo and effector proteins may be isolated from an organism by expression of tagged pAgo and/or effector proteins in said organism, and isolation of a protein complex comprising said pAgo and effector proteins on the basis of the tag. Said isolated protein complexes can be isolated using the tagged pAgo and/or effector proteins.

Said expression construct may be codon-optimised to enhance expression of the pAgo and effector proteins in a prokaryotic cell of interest, preferably *E*. *coli.*

Further optimization may include the removal of cryptic splice sites, removal of cryptic polyA tails and/or removal of sequences that may lead to unfavorable folding of the mRNA, for expression in eukaryotic cells. In addition, the expression construct may encode a protein export signal for secretion of the pAgo and effector proteins out of the cell into the periplasm of prokaryotes, allowing efficient purification of the pAgo and effector proteins.

Methods for purification of pAgo proteins and effector proteins are known in the art and are generally based on chromatography such as affinity chromatography, ion exchange chromatography and size exclusion chromatography, to remove contaminants. In addition to contaminants, it may also be necessary to remove undesirable derivatives of the product itself such as degradation products and aggregates. Suitable purification process steps are provided in Berthold and Walter, 1994 (Berthold and Walter, 1994. Biologicals 22: 135- 150).

As an alternative, or in addition, a recombinant pAgo and/or effector protein may be tagged with one or more specific tags by genetic engineering to allow attachment of the protein to a column that is specific to the tag and therefore be isolated from impurities. The purified protein is then exchanged from the affinity column with a decoupling reagent. The method has been routinely applied for purifying recombinant protein. Conventional tags for proteins, such as histidine tag, are used with an affinity column that specifically captures the tag (e.g., a Ni-IDA column for the histidine tag) to isolate the protein from other impurities. The protein is then exchanged from the column using a decoupling reagent according to the specific tag (e.g., imidazole for histidine tag). This method is more specific, when compared with traditional purification methods.

Suitable tags include histidine tag, c-myc domain (EQKLISEEDL), hemagglutinin tag (YPYDVPDYA), maltose-binding protein, glutathione-S-transferase, FLAG tag peptide, biotin acceptor peptide, streptavidin-binding peptide and calmodulin-binding peptide, as presented in Chatterjee, 2006 (Chatterjee, 2006. Cur Opin Biotech 17, 353-358). Methods for employing these tags are known in the art and may be used for purifying a pAgo protein and effector protein. A pAgo and effector protein may be tagged with one or more tags such as, for example, a histidine tag and a maltose-binding protein.

Said one or more tags may be separated from the pAgo protein and/or effector protein by a protease cleavage site, allowing removal of the tag or tags from the pAgo and/or effector protein after purification. Said removal may be followed by a separation step in which the pAgo and/or effector protein is purified from the tag or tags and from the protease. A preferred protease cleavage site is provided by the amino-acid sequence Glu-Asn-Leu-Tyr-Phe-Gln-(Gly/Ser), which amino acid sequence is recognized by a Tobacco Etch Virus (TEV) protease.

Methods for expression proteins in *E. coli* are known in the art and can be used for expression and purification of the pAgo and effector proteins.

In a preferred method, pAgo and/or effector proteins are expressed in *E. coli.* The expression plasmid is transformed into *E*. *coli,* for example into strain BL21 Star (DE3). Following growth at 37 °C in a desired culture volume, until an OD_{600 nm} of ~0.25, the culture is incubated at 18 °C after which isopropyl β-D-1-thiogalactopyranoside is added to a final concentration of 0.25 mM. The culture is then further incubated at 18 °C overnight. The cells are harvested and lysed for example in NiNTA lysis buffer (500 mM NaCl, 20 mM Imidazole, 20 mM HEPES pH 7.5, 4-benzenesulfonyl fluoride hydrochloride (AEBSF) 0.1 mg/mL, Pepstatin A 1 µg/mL) and stored at -80 °C prior to protein purification. The lysate may subsequently be spun, for example at 30.000 x g for 30 min. The cleared lysate may be filtered and run over a nickel resin column, for example by using a HisTrap HP column (Cytiva Life Sciences)). After washing the column with a NiNTA wash buffer, for example 500 mM NaCl, 5 mM Imidazole, 20 mM Tris pH 8, until no more protein is present in the flow through, the protein of interest may be eluted using a NiNTA elution buffer, for example 500 mM NaCl, 350 mM Imidazole, 20 mM Tris-HCl, pH 8.

If required, the resulting pAgo and/or effector protein may be further purified over a second column, for example an amylose column, following which the protein is cleaved from the affinity tag or tags by addition of a protease such as a TEV protease. If required, an additional chromatography step, or steps, such as a size exclusion chromatography step and/or affinity chromatography step, may be added to separate the tag from the pAgo protein and/or effector, and/or to achieve higher purity.

Hybridization of the gNA to a target nucleic acid molecule by base pairing will result in activation of the detection system for detecting a target nucleic acid molecule according to the invention. Said activated detection system will catalyze conversion of a nicotinamide adenine dinucleotide, or analogue thereof, yielding nicotinamide (NAM) and adenosine-diphosphate-ribose (ADPR), or an analogue or analogues thereof. Said conversion may be determined by any method known in the art, including high pressure liquid chromatography (HPLC), mass spectrometry (MS), gas chromatography (GC), photometric detection, colorimetric detection, bioluminescent detection, fluorescent detection, and combinations thereof such as nuclear magnetic resonance (NMR), HPLC-MS, HPLC-MS-MS, GC-MS and GC-MS-MS. Suitable methods and kits include the colorimetric NAD/NADH Assay Kit (Abcam, Cambridge, Massachusetts), EnzyChrom^{™} NAD/NADH Assay Kit (Hayward, California), NAD/NADH-Glo^{™} Assay kit (Promega, Madison, Wisconsin), NAD/NADH Cell-Based Assay Kit (Cayman Chemical, Ann Arbor, Michigan, USA), NAD/NADH Quantitation Kit (Sigma Aldrich, Saint Louis, USA), (NADP/NADPH Quantitation Kit (Sigma Aldrich) and Fluoro NAD (CellTechnology, Hayward California).

As an alternative, or in addition, conversion of nicotinamide adenine dinucleotide may be monitored indirectly, for example by monitoring deacetylation of a p53 substrate sequence Gln-Pro-Lys-Lys(ε-acetyl)-AMC, for which NAD+ acts as a co-substrate. Deacetylation may be quantified with an excitation wavelength of 350-360 nm and an emission wavelength of 450-465 nm (Direct Fluorescent Screening Assay Kit, Cayman Chemical).

The conversion of nicotinamide adenine dinucleotide is preferably monitored using a NAD analogue that becomes fluorescent upon conversion of the nicotinamide adenine dinucleotide, such as nicotinamide 1,N6-ethenoadenine dinucleotide (ε-NAD+), β- nicotinamide- N⁶- (2- (6- [fluoresceinyl]aminohexanoyl) aminoethyl)adenine dinucleotide (6-Fluo-10-NAD⁺), or β- Nicotinamide- 1, N⁶-ethenoadenine dinucleotide phosphate ( ε-NADP⁺).

Said detection system preferably is provided in a receptacle to which a sample comprising nucleic acid molecules may be added. The term receptacle, as is used herein, refers to a container that holds, or can hold, said detection system and the sample comprising nucleic acid molecules. Said receptacle is, for example, a tube, such as an Eppendorf tube, or a well of a multiwell plate such as a 24 well plate, a 96 well plate, a 384 well plate or a 1024 well plate. Said receptacle preferably comprises the short pAgo protein, effector protein, guide nucleic acid (gNA) and, optionally a nicotinamide adenine dinucleotide (NAD) or an analogue thereof, in a dried, preferably lyophilized, form. Said receptacle may further comprise a suitable buffer and/or salts. As an alternative, said suitable buffer and/or salts may be provided together with the sample comprising nucleic acid molecules.

The NADase reaction, i.e. the conversion of a nicotinamide adenine dinucleotide (NAD⁺), or an analogue thereof, to nicotinamide (NAM) and adenosine diphosphate ribose (ADPR), or the corresponding analogue or analogues thereof, is preferably performed at a temperature between 20 °C and 75 °C, preferably between 37 °C and 70 °C, preferably between 50 °C and 65 °C. A person skilled in the art will appreciate that the optimal temperature will depend on the organism or organisms from which the pAgo protein and effector protein comprising either TIR-APAZ domains or SIR2-APAZ domains, are obtained. For example, proteins from organisms such as *Maribacter polysiphoniae* will have a lower temperature optimum, when compared to organisms such as *Crenotalea thermophila.* In general, the temperature optimum for organisms such as *M. polysiphoniae* is between 20 °C and 60 °C, such as between 37 °C and 55 °C, while the temperature optimum for organisms such as *C*. *thermophila* is between 45 °C and 75 °C, such as between 55 °C and 65 °C.

The NADase reaction is preferably performed at an ionic strength of 10-1000 millimole/L, preferably at an ionic strength of 20-500 millimole/L. Said ionic strength preferably is provided by a buffer or buffers and/or a salt or salts.

The NADase reaction is preferably performed at a pH between 5 and 9, preferably between 5.5 and 7.5, such as a pH between 6 and 7, including pH 6.5. Said pH preferably is kept constant in the present of a buffering substance, such as bis-tris propane (BTP), 2-(N-morpholino)ethanesulfonic acid (MES), 4-(2-hydroxyethyl)-1-piperazineethanesulfonic acid (HEPES), citric acid, or mixtures thereof.

The NADase reaction is preferably performed in the presence of 20 - 500 mM salt, preferably 50 - 250 mM salt, such as 100 - 150 mM salt, including about 125 mM salt. Said salt preferably comprises a monovalent salt such as a sodium salt or a potassium salt, and/or a divalent salt such as a manganese and/or a magnesium salt. Said salt preferably comprises a monovalent salt and a divalent salt, more preferably a potassium salt and a magnesium salt, preferably potassium chloride and magnesium chloride. The amount of a monovalent salt such as potassium chloride is preferably about 20-100 times higher than the amount of a divalent salt such as magnesium chloride.

Said receptacle may further include means for a pre-amplification step, preferably a asymmetric amplification step, preferably means for a polymerase chain reaction (PCR) or for an asymmetric isothermal amplification step such as strand displacement amplification (SDA), nucleic acid sequence-based amplification (NASBA), or loop-mediated isothermal amplification (LAMP), including suitable primers and strand displacing polymerase such as phi29 DNA polymerase (New England Biolabs #M0269), or variants thereof such as QualiPhi^{®} DNA Polymerase (4basebio, Madrid, Spain).

A preferred detection system according to the invention provides a binary outcome such as a lateral flow assay (LFA). The principle of LFA is that a reaction product, in the present case NAD or an analogue thereof, is immobilized at a first point, termed control line, while a converted product is immobilized at a second point, termed test line. Said converted product may be nicotinamide (NAM), adenosine-diphosphate-ribose (ADPR), or an analogue thereof. An LFA is typically composed of a nitrocellulose membrane, sample pad, conjugate pad, wicking or absorbent pad and backing pad (Jauset-Rubio et al., 2016. Sci Rep 6: 37732). Nitrocellulose membranes are most commonly used as they facilitate a support capable of use for both reaction and detection. Capture biomolecules, for example antibodies, are preferably deposited on the nitrocellulose to form the test and control lines via a combination of electrostatic interactions, hydrogen bonds and/or hydrophobic interactions (Jauset-Rubio et al., 2016. Sci Rep 6: 37732).

It will be clear to a person skilled in the art LFA may employ molecules such as single heavy chain variable domain antibodies, and variants and derivatives thereof, including scFv, tandem scFv, scFab, and improved scFab (Koerber et al., 2015. J Mol Biol 427: 576-86), chimeric variants of monoclonal and single heavy chain variable domain antibodies. In addition, antibody mimetics such as a designed ankyrin repeat protein (i.e. DARPIN), a binding protein that is based on a Z domain of protein A, a binding protein that is based on a fibronectin type III domain (i.e. Centyrin), engineered lipocalin (i.e. anticalin), human IgG CH2 domain based binding proteins (i.e. Abdurin), human IgG CH3 domain based binding proteins (i.e. Fcab), and a binding protein that is based on a human Fyn SH3 domain ((i.e. Fynomer) (Skerra, 2007. Current Opinion Biotechnol 18: 295-304; Škrlec et al., 2015. Trends Biotechnol 33: 408-418), may be used for capturing NAD or an analogue thereof at a control line, and nicotinamide (NAM), adenosine-diphosphate-ribose (ADPR), or an analogue or analogues thereof at a test line.

### Methods of detecting and/or quantifying a target nucleic acid molecule

In an embodiment, the invention provides a method of detecting and/or quantifying a specific nucleic acid molecule in a sample comprising nucleic acid molecule, preferably comprising single stranded nucleic acid molecule, the method comprising the steps of contacting said sample with a short pAgo protein, comprising a MID and a PIWI domain; an effector protein comprising Toll/Interleukin1 receptor (TIR)-Analog of PAZ (APAZ) domains, or comprising SIR2-APAZ domains; a guide nucleic acid (gNA) that is able to hybridize to the nucleic acid molecule; and nicotinamide adenine dinucleotide or analogue thereof, preferably with a detection system according to the invention, and detecting conversion of said nicotinamide adenine dinucleotide or analogue thereof.

Said short pAgo proteins comprising a MID and a PIWI domain, and effector protein comprising TIR-APAZ domains or SIR2-APAZ domains, are preferably provided in a molar ratio of between 5:1 and 1:5, preferably between 3:1 and 1:3, preferably between 2:1 and 1:2, preferably about 1:1.

Said sample comprising nucleic acid molecule preferably comprises DNA, including copy DNA (cDNA). If necessary, nucleic acid material, including viral DNA or viral RNA, may be purified from a sample using, for instance, a combination of physical and chemical methods. Commercially available systems for nucleic acid isolation are preferably used, such as the NucliSENS^{®} easyMAG^{®} or NucliSENS^{®} miniMAG^{®} nucleic acid extraction system (bioMérieux, Marcy l'Etoile, France), or a MagNA Pure 96 System (Roche Diagnostics, Almere, The Netherlands).

RNA may be isolated from a sample by any technique known in the art, including but not limited to suitable commercial RNA isolation kits such as Trizol (Invitrogen; Carlsbad, California), RNAqueous^{®} (Applied Biosystems/Ambion, Austin, Tx), Qiazol^{®} (Qiagen, Venlo, The Netherlands), Agilent Total RNA Isolation Lits (Agilent; Santa Clara, California), RNA-Bee^{®} (Tel-Test. Friendswood, Texas), the RNeasy mini kit (Qiagen, Venlo, The Netherlands), and Maxwell^{™} 16 Total RNA Purification Kit (Promega; Madison, Wisconsin).

Isolated RNA is preferably reverse transcribed with the aid of a RNA-dependent DNA polymerase into single or double stranded copy DNA (cDNA), using methods known to a person skilled in the art. Reverse transcription may be primed by universal primers, such as random hexamers or nonamers, or by one or more specific primers, such as primers that specifically hybridize to a nucleic acid from a gene or a pathogen.

DNA, including genomic DNA, may be isolated from a sample by any technique known in the art, including but not limited to suitable commercial DNA isolation kits such as Quick-DNA Viral Kits (Zymo Research; Irvine, CA), NucleoSpin Dx Virus (Macherey-Nagel, Düren, Germany), QIAamp DNA Blood Mini Kit (Qiagen, Venlo, The Netherlands), and EasyPure^{®} Viral DNA/RNA Kit (Transgen Biotech, Beijing, China).

The resulting DNA, including cDNA can be used directly in a detection system according to the invention. Said DNA may be amplified prior to detection to increase detection levels. Amplification may be performed by any suitable amplification system including, for example, ligase chain reaction (LCR), an isothermal amplification method such as nucleic acid sequence-based amplification (NASBA), cleavage-based signal amplification of RNA (Zhao et al., 2013. Nature Comm 4: 1493), transcription mediated amplification, strand displacement amplification or polymerase chain reaction (PCR), preferably an isothermal amplification method.

Said amplification may be mediated by an isothermal reaction, preferably a single tube, isothermal amplification reaction. Suitable isothermal amplification reactions include transcription mediated amplification (TMA; WO1991001384A1), Nucleic Acid Sequence Based Amplification (NASBA; US5654142A), strand displacement amplification (US5712124A), Rolling Circle Amplification (RCA; US5714320A), loop-mediated isothermal amplification (LAMP; Notomi et al., 2000. Nucleic Acids Res 28: 63e; WO2000/028082), Recombinase Polymerase Amplification (RPA) reaction (WO2003072805), Helicase-Dependent Amplification (HAD; WO2004027025A2) and Nicking and Extension Amplification Reaction (NEAR; WO2009012246), and variants thereof.

A preferred isothermal amplification reaction is provided by loop-mediated isothermal amplification (LAMP). Recently, RT-LAMP technology was combined with Cas12 detection of DNA to generate a high throughput COVID-19 detection test (Broughton et al., 2020. Nature Biotech 10.1038/s41587-020-0513-4; Chen et al., 2018. Science 360: 436-439).

LAMP normally employs six primers that recognize distinct target sequences on a template nucleic acid strand. Four of these primers are termed "inner primers", termed LF, LB, FIP and BIP, which are designed to result in the generation of novel DNA strands. The outer primers, termed F3 and B3, anneal to a template strand and also generate new DNA. These primers are accompanied by a DNA polymerase which aids in strand displacement and releases the newly formed DNA strands. This amplification takes place in less than one hour such as in less than 20 minutes, under isothermal conditions between 60-65 °C.

Amplification may generate a single stranded target nucleic acid molecule, such as asymmetric amplification, rolling circle amplification, or may be followed by a method to generate a single stranded target nucleic acid molecule, such as conventional melting and/or alkali treatment enzymatic processing such as helicase-mediated unwinding of the DNA, selective enzymatic digestion of one of the two strands from a double stranded nucleic acid product, and immobilization of a double stranded DNA molecule, for example an amplification product, of which one of the strands is labeled, for example biotinylated, followed by separation of the labeled and non-labeled strands. As is shown in the Examples, pre-amplification, followed by the generation of single stranded target nucleic acid molecules, may decrease the limit of detection (LoD) of a double stranded target nucleic acid molecule down to the attomolar level (see also Figure 8).

The step of contacting a sample comprising a target nucleic acid molecule, or suspected to comprise a target nucleic acid molecule, with short pAgo and effector proteins and a gNA is preferably performed at a temperature between 20 °C and 75 °C, preferably between 37 °C and 70 °C, preferably between 50 °C and 65 °C, more preferably between 60-65 °C.

If an amplification step is included prior to step of contacting a sample comprising a target nucleic acid molecule, or suspected to comprise a target nucleic acid molecule, with pAgo and effector proteins and a gNA, the amplification and contacting step are preferably performed in one and the same reaction tube, preferably in a one-step/one-pot reaction. Said amplification reaction, preferably an isothermal reaction such as LAMP or RPA, preferably is integrated with the pAgo-based nucleic acid detection system of the invention as a "one pot" reaction system. An advantage of such one pot, or single tube, system is a reduced risk for contamination of the samples, or cross-contamination of different samples.

Conversion of a nicotinamide adenine dinucleotide or analogue thereof to nicotinamide (NAM) and adenosine diphosphate ribose (ADPR), or the corresponding analogue or analogues thereof, may be determined, preferably quantified, by any method known in the art. Preferred methods include thin-layer chromatography (Heard, 1983. Anal Biochem 130: 185-188), photometric detection, colorimetric detection, bioluminescent detection, fluorescent detection, and combinations thereof as are employed in a lateral flow assay such as a lateral flow immunoassay. Said conversion of nicotinamide adenine dinucleotide is preferably determined by a colorimetric method or a fluorescent method.

A most preferred method comprises the use of a NAD analogue, for example an analogue that becomes fluorescent upon conversion of the nicotinamide adenine dinucleotide, such as nicotinamide 1,N⁶-ethenoadenine dinucleotide (ε-NAD⁺), β-nicotinamide- N⁶- (2- (6- [fluoresceinyl]aminohexanoyl)aminoethyl) adenine dinucleotide (6-Fluo-10-NAD⁺), and β- nicotinamide- 1, N⁶-ethenoadenine dinucleotide phosphate ( ε-NADP⁺). The amount of converted nicotinamide adenine dinucleotide or analogue thereof, or the relative amount when compared to a reference sample, may be used for quantification of the target nucleic acid molecule in the sample. Said reference is a sample that is known not to comprise the target nucleic acid molecule, or a sample that comprises a known amount of the target nucleic acid molecule.

Conversion of such NAD analogue may be monitored and quantified using, for example, a fluorescence reader such as a fluorescence microplate reader such as a Synergy Neo2 plate reader (BioTek, Winooski, USA), or a SpectraMax Gemini^{™} XPS/EM Microplate Reader (Molecular Devices, San Jose, Calfornia, USA).

The methods of the invention for detecting and/or quantifying specific nucleic acid sequences may be used in human healthcare, veterinary diagnostics, detection of plant pathogens, detection of water contaminants and the detection of food and feed contaminants. In addition, the methods of the invention for detecting specific nucleic acid sequences may be used for detecting beneficial organisms. In general, the methods of the invention can be used for detection and/or quantification of bacterial, fungal, archaeal, algal, protozoal, eukaryotic, viral and viroidal pathogens. In addition, the methods of the invention may also be used for detection and diagnosis of genetic alterations or traits in human, animals and plants that result from alterations in the genome of these organisms, or that result from alterations in RNA expression products. In a preferred method of the invention, the sample comprises nucleic acid molecules from a pathogen, or is suspected to comprise nucleic acid molecules from a pathogen, including a human, veterinary, or plant pathogen.

For example, methods of the invention may be used to detect presence or absence of an altered target nucleotide sequence such as a single nucleotide polymorphism (SNP), and/or for quantification of said SNP. Said SNP may comprise a natural or non-mutated sequence and a variant thereof in which one nucleotide at a specific position, or a few nucleotides, such as two or three nucleotides, at specific positions are altered. For example, a gNA may be selected that enables discrimination of a single base change in a target nucleic acid molecule. For this, one or more additional alterations may be provided in the gNA that are not caused by a nucleotide variant of the target nucleic acid molecule, but that are introduced to strengthen the discrimination between two variants of a target nucleic acid molecule. As an alternative, or in addition, point mutations may be introduced, for example in the short pAgo MID domain, to render the interaction with a gNA-target nucleic acid molecule either more, or less sensitive, and/or rendering activation of the resulting short pAgo-effector protein complex more or less sensitive to mismatches. As an alternative, or in addition, buffer conditions may be varied that render the interaction with a gNA-target nucleic acid molecule either more, or less sensitive, and/or rendering activation of the resulting short pAgo-effector protein complex more or less sensitive to mismatches.

### EXAMPLES

### Example 1

### Materials and methods

### Plasmid construction

Genes expressing short pAgo systems or individual proteins thereof were amplified from synthetic DNA constructs ordered from Twist Bioscience (South San Francisco, USA). Sequences are provided in Figure 2. The genes were cloned in the pET-His6-MBP-TEV-LIC cloning vector (Addgene, Watertown, Massachusetts, USA). This places the genes under control of a T7 promoter and allowed for expression of the proteins that are linked to an N-terminal 6x-His-Maltose Binding Protein (MBP) tag by a Tobacco Etch Virus (TEV) cleavage site-containing linker.

### Protein purification

Individual proteins and complexes with N-terminal 6x-his-MBP-tags were heterologously expressed in *E. coli* BL21 Star (DE3; ThermoFisher Scientific, Waltham, Massachusetts, USA). A single colony was used to inoculate a 10 mL LB culture which was incubated o/n at 37 °C. The o/n culture was used to inoculate 8x750 mL LB medium which were incubated at 37 °C. When an OD_{600 nm} of ~0.25 was reached, the temperature of the incubator was switched to 18 °C. After about 1 h (when OD_{600 nm} reached ~0.6), protein expression was induced by addition of IPTG to a final concentration of 0.25 mM. Protein expression took place at 18 °C for 16 hours. Cells were harvested by centrifugation at 5000 x g at 4 °C for 15 min. Cells were resuspended in NiNTA lysis buffer (500 mM NaCl, 20 mM Imidazole, 20 mM HEPES pH 7.5, 4-benzenesulfonyl fluoride hydrochloride (AEBSF) 0.1 mg/mL, Pepstatin A 1 µg/mL) and stored at -80 °C prior to protein purification. After thawing, cells were lysed by sonication (QSONICA Q700A-220 sonicator with ½ inch tip; amp 50%, 1s ON/2s OFF, 5 minutes total ON time) and the lysate was centrifuged for 30 min at 30,000 x g at 4 °C. The clarified lysate was applied to a 5 mL HisTrap HP column (Cytiva Life Sciences, Marlborough, Massachusetts, USA). The column was washed with ~6 column volumes (CV) NiNTA buffer A (500 mM NaCl, 5 mM Imidazole, 20 mM Tris pH 8) or until A_{280 nm} of the eluate was below 250 a.u.. The protein was eluted in NiNTA buffer B (500 mM NaCl, 350 mM Imidazole, 20 mM Tris pH 8). Fractions containing the protein of interest were pooled, and loaded on a 20 mL Amylose resin (New England Biolabs) column. The column was washed with ~3x CV Amylose wash buffer (500 mM NaCl, 1 mM DTT, 20 mM Tris pH 8) or until the A_{280 nm} was ~0 a.u.. The protein was eluted in Amylose elution buffer (Amylose Wash Buffer supplemented with 20 mM maltose).

Fractions containing the protein of interest were pooled, EDTA was added to a final concentration of 2 mM, and TEV protease was added in a 1:50 (w/w) ratio. The sample was dialyzed against dialysis buffer (250 mM KCl, 1 mM DTT, 2 mM EDTA, 20 mM HEPES pH 7.5) for 16 h. Cleavage of the MBP tag by TEV was confirmed by SDS-PAGE and Coomassie Brilliant Blue (CBB) staining. The sample was diluted 1:1 with 20 mM HEPES pH 7.5 and loaded on a HiTrap Heparin column (Cytiva Life Sciences). The column was washed with 99.7% ion-exchange buffer I (100 mM KCl, 1 mM DTT, 8 mM HEPES pH 7.5) and 1.3% ion-exchange buffer II (2.5 M KCl, 1 mM DTT, 20 mM HEPES pH 7.5) until the A_{280 nm} was about 0. To protein was eluted with ion-exchange buffer II by applying a gradient from 1.3% to 50% over a total volume of 60 mL. Peak fractions were analyzed by SDS-PAGE and CBB staining and fractions containing the protein of interest were combined. The sample was concentrated to ~1 mL using centrifugal filter units (Amicon; Merck KGaA, Darmstadt, Germany) according to the protocol of the manufacturer. The sample was centrifuged for 10 min at 16,000 x g at 4°C and the protein was fractionated on a custom 200 mL Superdex 200 Prep Grade resin (Cytiva Life Sciences) column, eluting with SEC buffer. Peak fractions were analyzed by SDS-PAGE and CBB staining and fractions containing the protein of interest were combined. The sample was concentrated to the desired concentration using centrifugal filter units (Amicon) according to the protocol of the manufacturer. Samples were aliquoted, flash frozen in liquid nitrogen, and stored at -80°C until further use.

### ε-NAD⁺ assays

A reaction mixture of purified protein complexes in SEC buffer, ε-NAD⁺, RNA guide, and 5X reaction buffer (50 mM MES pH 6.5, 375 mM KCl, and 10 mM MgCl2) was prepared on ice in 96-well plates. The mixture was incubated at room temperature for 15 min, after which DNA target was added to a final concentration of 200 nM unless otherwise indicated. RNA guide and ssDNA target sequences used for these experiments are provided in Table 1. Final concentrations of each component were 1 µM SPARTA complex, 25 µM ε-NAD⁺ (nicotinamide 1,N6-ethenoadenine dinucleotide), 10 mM MES pH 6.5, 125 mM KCl, and 2 mM MgCl2 in a final volume of 60 µL. After addition of the target DNA, the 96-well plate was transferred to a preheated BioTek Synergy Neo2 plate reader (55 °C for CrtSPARTA and 37 °C for MapSPARTA unless specified otherwise). Fluorescence intensity was measured in kinetic mode using an excitation wavelength of 310 nm and emission wavelength of 410 nm. All experiments were performed in triplicates and error bars indicate standard deviation.

For pH-range experiments, reaction mixtures were prepared as described above, but instead of 10 mM MES pH 6.5, each sample contained a final concentration of 10 mM buffer mix (BTP, MES, and citric acid in a 1:1:1 molar ratio) with a pH in the range of 4.0 to 9.5. For optimum temperature determination, reaction mixtures were incubated at the indicated temperatures for 60 min, and end-point fluorescence intensity was measured using excitation of wavelength of 310 nm and emission wavelength of 410 nm using a BioTek Synergy Neo2 plate reader. All experiments were performed in triplicates and error bars indicate standard deviation.

### Results

Various CRISPR-Cas systems (Gootenberg et al., 2017. Science 356: 438-442; Chen et al., 2018. Science 360: 436-439; Gootenberg et al., 2018. Science 360: 439-444; Li et al., 2019. ACS Synth Biol 8: 2228-2237; Steens et al., 2021. bioRxiv: 429135) as well as long pAgo proteins (Song et al., 2019. bioRxiv: 491738; Wang et al., 2020. Biosens Bioelectron 177: 112932) have been repurposed as reprogrammable nucleic acid detection tools. As also SPARTA systems can be reprogrammed to recognize ssDNA molecules with any given sequence, we hypothesized that SPARTA systems could be used for the detection of nucleic acids. To determine if target recognition sensitivity and selectivity is comparable to that of established tools, we optimized reaction conditions and investigated mismatch sensitivity as well as the limit of detection of ssDNA targets. To facilitate high-throughput analysis of SPARTA activity, an analogue of NAD, nicotinamide 1,N6-ethenoadenine dinucleotide (ε-NAD⁺) was used as a substrate. Fluorescence of ε-NAD⁺ is quenched by its nicotinamide group and it is activated upon SPARTA-mediated conversion of ε-NAD⁺ into NAM and fluorescent ε-ADPR. This allows to directly monitor SPARTA activity by measuring fluorescence.

As is shown in Figure 3, CrtSPARTA is active at temperatures between 37 °C and 70 °C (optimum at 55 -65 °C), whereas MapSPARTA is active between 25 °C and 65 °C (optimum 50 °C) (Fig. 3). For both MapSPARTA and CrtSPARTA, activity was observed at a pH between 5.5 and 8.5, with an optimum activity at a pH between 6.0 and 6.5. The activity dropped at pH > 7.5 (Fig. 4). After optimization of the reaction buffer conditions, the limit of detection was determined by incubating the system with varied concentrations of target ssDNA. Both systems are able to detect target ssDNA in the low nM range. Extrapolation of these data indicates that detection of a target DNA may occur in the pM range, without pre-amplification (Fig. 5).

Next, we probed the guide requirements of the SPARTA systems. CrtSPARTA provided with 5'-phosphorylated RNA guides ranging in length from 17 to 30 nucleotides showed little variation in NADase activity in the presence of a complementary ssDNA target (Fig. 6). In contrast, reduced activity was observed when a 16-nucleotide or 15-nucleutide guide was provided and even more so when a 12-nucleotide guide was provided. Activity of MapSPARTA was the highest with 30-nucleotide guide and gradually decreased with shorter guides. The activity was completely abolished in the reactions with 12-nucleotide guide (Fig. 6).

To determine the specificity of nucleic acid detection, we introduced double- and single mismatches between the 21-nt guide RNA and a complementary target ssDNA. Single mismatches between guide RNA and target DNA at positions 5-10 from 5'-end of the guide attenuated the activation of CrtSPARTA (Fig. 7A,C). Mismatch sensitivity increased when double mismatches were introduced and spanned from positions 5-6 to 17-18. The activation of CrtSPARTA was almost completely abolished for targets with double mismatches at positions 5-6 to 9-10 and 14-15. In contrast, double mismatches at the 5'-end or 3'-end of the guide (positions 1-2 to 4-5 and 18-19 to 20-21) did not lower the NADase activity of CrtSPARTA.

Single guide-target mismatch sensitivity of MapSPARTA is the highest at positions 6, 11 and 15 (Fig. 7B). Double mismatches decreased the activity of MapSPARTA when they were located between positions 4-5 to 14-15, with the exception of a double mismatch at position 12-13, which did not lower the activity of MapSPARTA (Fig. 7D).

### Example 2

### Materials and methods

To determine a limit of detection for dsDNA targets, initial PCR amplification was followed by detection with MapSPARTA. For this, 35-cycle PCRs were performed either on colonies transformed with plasmid (pAP001; a pUC-based plasmid containing partial M13 gene IV and comprising sequence oBK090), or on purified plasmid (pAP001) in MilliQ H₂O. The concentration of purified plasmid ranged from nM to attoM (aM) and was determined using the Qubit^{™} dsDNA HS Assay Kit (ThermoFisher Scientific). Colonies were suspended in 20 µL MilliQ H₂O and incubated at 95 °C for 5 min. 50 µL PCR mixture contained 1 µL template, 25 µL Phusion High-Fidelity PCR Master Mix (ThermoFisher Scientific) and 0.5 µM of each primer (oAP030/oAP039). The first five 5' nucleotides of oAP039 contain phosphorothioate groups to protect that strand from degradation by T7 exonuclease. After PCR cleanup (New England Biolabs) dsDNA products were either melted at 95 °C for 10 min and mixed with gRNA (oBK084) to form RNA:DNA hybrids or were used directly as targets in MapSPARTA detection assays containing T7 exonuclease. SPARTA reaction mixtures were prepared as described above. Reactions with T7 exonuclease were performed in the presence of 5U T7 exonuclease (New England Biolabs). All dsDNA detection reactions were incubated at 37 °C. Fluorescence intensity was measured in kinetic mode every 2 minutes, using excitation wavelength of 310 nm and emission wavelength of 410 nm in a Synergy Neo2 or SH1M2FG plate reader (Biotek).

Results As is shown in Figure 8, double stranded target DNA can be detected in the attomolar range using a detection system according to the invention after pre-amplification of the double stranded target DNA and conversion of the amplified double stranded target DNA into a single strand.

### Example 3

### Materials and methods

Individual pAgo and effector proteins from *Bacillales bacterium* (Bab), *Elioreae tepidiphila* (Elt) and *Parabacteroides distasonis* (Pad) were generated in *E. coli* and purified as described in Example 1. Assay and assay conditions were as described in Example 1. Reaction temperature was 37 °C.

Results As is shown in Figure 9, pAgo and effector proteins from *Bacillales bacterium, Elioreae tepidiphila* and *Parabacteroides distasonis* are active and can be used in a detection system according to the invention.

### Example 4

### Materials and methods

Individual pAgo and effector proteins from *Bacillales bacterium* (Bab) were generated in *E. coli* and purified as described in Example 1. Assay and assay conditions were as described in Example 1.
Results As is shown in Figure 10, pAgo and effector proteins from *Bacillales bacterium* are active at temperatures between 37 °C and 70 °C (optimum at 55 -60 °C).

**Table 1. RNA guide and ssDNA target sequences used for in vitro SPARTA experiments.**

| ***Name*** | ***Sequence (5'-3' orientation)*** | ***Description*** |
|---|---|---|
| *oBK084* | P-UGACGGCUCUAAUCUAUUAGU | 21nt RNA guide |
| *oBK090* | TATCTTTAGGAGCACTAACAACTAATAGATTA GAGCCGTCAATAGATAATACATTTGAGG | 60nt ssDNA target |
| *oBK215* | P-UGACGGCUCUAAUCUAUUAGUUGUUAGUGC | 30nt RNA guide |
| *oBK216* | P-UGACGGCUCUAAUCUAUUAGUUGUU | 25nt RNA guide |
| *oBK217* | P-UGACGGCUCUAAUCUAUU | 18nt RNA guide |
| *oBK391* | P-UGACGGCUCUAAUCUAU | 17nt RNA guide |
| *oBK392* | P-UGACGGCUCUAAUCUA | 16nt RNA guide |
| *oBK218* | P-UGACGGCUCUAAUCU | 15nt RNA guide |
| *oBK219* | P-UGACGGCUCUAA | 12nt RNA guide |
| *oBK221* | TATCTTTAGGAGCACTAACAACTAATAGATTA GAGCCGTacATAGATAATACATTTGAGG | oBK090 mismatch 1-2 |
| *oBK222* | TATCTTTAGGAGCACTAACAACTAATAGATTA GAGCCGgaAATAGATAATACATTTGAGG | oBK090 mismatch 2-3 |
| *oBK223* | TATCTTTAGGAGCACTAACAACTAATAGATTA GAGCCtgCAATAGATAATACATTTGAGG | oBK090 mismatch 3-4 |
| *oBK224* | TATCTTTAGGAGCACTAACAACTAATAGATTA GAGCatTCAATAGATAATACATTTGAGG | oBK090 mismatch 4-5 |
| *oBK225* | TATCTTTAGGAGCACTAACAACTAATAGATTA GAGaaGTCAATAGATAATACATTTGAGG | oBK090 mismatch 5-6 |
| *oBK226* | TATCTTTAGGAGCACTAACAACTAATAGATTA GAtaCGTCAATAGATAATACATTTGAGG | oBK090 mismatch 6-7 |
| *oBK227* | TATCTTTAGGAGCACTAACAACTAATAGATTA GctCCGTCAATAGATAATACATTTGAGG | oBK090 mismatch 7-8 |
| *oBK228* | TATCTTTAGGAGCACTAACAACTAATAGATTA tcGCCGTCAATAGATAATACATTTGAGG | oBK090 mismatch 8-9 |
| *oBK229* | TATCTTTAGGAGCACTAACAACTAATAGATTc tAGCCGTCAATAGATAATACATTTGAGG | oBK090 mismatch 9-10 |
| *oBK230* | TATCTTTAGGAGCACTAACAACTAATAGATgc GAGCCGTCAATAGATAATACATTTGAGG | oBK090 mismatch 10-11 |
| *oBK231* | TATCTTTAGGAGCACTAACAACTAATAGAggA GAGCCGTCAATAGATAATACATTTGAGG | oBK090 mismatch 11-12 |
| *oBK232* | TATCTTTAGGAGCACTAACAACTAATAGcgTA GAGCCGTCAATAGATAATACATTTGAGG | oBK090 mismatch 12-13 |
| *oBK233* | TATCTTTAGGAGCACTAACAACTAATAtcTTA GAGCCGTCAATAGATAATACATTTGAGG | oBK090 mismatch 13-14 |
| *oBK234* | TATCTTTAGGAGCACTAACAACTAATctATTA GAGCCGTCAATAGATAATACATTTGAGG | oBK090 mismatch 14-15 |
| *oBK235* | TATCTTTAGGAGCACTAACAACTAAgcGATTA GAGCCGTCAATAGATAATACATTTGAGG | oBK090 mismatch 15-16 |
| *oBK236* | TATCTTTAGGAGCACTAACAACTAcgAGATTA GAGCCGTCAATAGATAATACATTTGAGG | oBK090 mismatch 16-17 |
| *oBK237* | TATCTTTAGGAGCACTAACAACTccTAGATTA GAGCCGTCAATAGATAATACATTTGAGG | oBK090 mismatch 17-18 |
| *oBK238* | TATCTTTAGGAGCACTAACAACgcATAGATTA GAGCCGTCAATAGATAATACATTTGAGG | oBK090 mismatch 18-19 |
| *oBK239* | TATCTTTAGGAGCACTAACAAagAATAGATTA GAGCCGTCAATAGATAATACATTTGAGG | oBK090 mismatch 19-20 |
| *oBK240* | TATCTTTAGGAGCACTAACAcaTAATAGATTA GAGCCGTCAATAGATAATACATTTGAGG | oBK090 mismatch 20-21 |
| *oBK241* | TATCTTTAGGAGCACTAACAACTAATAGATTA GAGCCGTC[?]ATAGATAATACATTTGAGG | oBK090 mismatch 1 |
| *oBK242* | TATCTTTAGGAGCACTAACAACTAATAGATTA GAGCCGTaAATAGATAATACATTTGAGG | oBK090 mismatch 2 |
| *oBK243* | TATCTTTAGGAGCACTAACAACTAATAGATTA GAGCCGgCAATAGATAATACATTTGAGG | oBK090 mismatch 3 |
| *oBK244* | TATCTTTAGGAGCACTAACAACTAATAGATTA GAGCCtTCAATAGATAATACATTTGAGG | oBK090 mismatch 4 |
| *oBK245* | TATCTTTAGGAGCACTAACAACTAATAGATTA GAGCaGTCAATAGATAATACATTTGAGG | oBK090 mismatch 5 |
| *oBK246* | TATCTTTAGGAGCACTAACAACTAATAGATTA GAGaCGTCAATAGATAATACATTTGAGG | oBK090 mismatch 6 |
| *oBK247* | TATCTTTAGGAGCACTAACAACTAATAGATTA GAtCCGTCAATAGATAATACATTTGAGG | oBK090 mismatch 7 |
| *oBK248* | TATCTTTAGGAGCACTAACAACTAATAGATTA GcGCCGTCAATAGATAATACATTTGAGG | oBK090 mismatch 8 |
| *oBK249* | TATCTTTAGGAGCACTAACAAagAATAGATTA GAGCCGTCAATAGATAATACATTTGAGG TATCTTTAGGAGCACTAACAcaTAATAGATTA GAGCCGTCAATAGATAATACATTTGAGG TATCTTTAGGAGCACTAACAACTAATAGATTA GAGCCGTCcATAGATAATACATTTGAGG TATCTTTAGGAGCACTAACAACTAATAGATTA GAGCCGTaAATAGATAATACATTTGAGG TATCTTTAGGAGCACTAACAACTAATAGATTA GAGCCGgCAATAGATAATACATTTGAGG TATCTTTAGGAGCACTAACAACTAATAGATTA GAGCCtTCAATAGATAATACATTTGAGG TATCTTTAGGAGCACTAACAACTAATAGATTA GAGCaGTCAATAGATAATACATTTGAGG TATCTTTAGGAGCACTAACAACTAATAGATTA GAGaCGTCAATAGATAATACATTTGAGG TATCTTTAGGAGCACTAACAACTAATAGATTA GAtCCGTCAATAGATAATACATTTGAGG TATCTTTAGGAGCACTAACAACTAATAGATTA GcGCCGTCAATAGATAATACATTTGAGG TATCTTTAGGAGCACTAACAACTAATAGATTA tAGCCGTCAATAGATAATACATTTGAGG TATCTTTAGGAGCACTAACAACTAATAGATTc GAGCCGTCAATAGATAATACATTTGAGG TATCTTTAGGAGCACTAACAACTAATAGATgA GAGCCGTCAATAGATAATACATTTGAGG TATCTTTAGGAGCACTAACAACTAATAGAgTA GAGCCGTCAATAGATAATACATTTGAGG TATCTTTAGGAGCACTAACAACTAATAGcTTA GAGCCGTCAATAGATAATACATTTGAGG TATCTTTAGGAGCACTAACAACTAATAtATTA GAGCCGTCAATAGATAATACATTTGAGG TATCTTTAGGAGCACTAACAACTAATcGATTA GAGCCGTCAATAGATAATACATTTGAGG TATCTTTAGGAGCACTAACAACTAAgAGATTA GAGCCGTCAATAGATAATACATTTGAGG TATCTTTAGGAGCACTAACAACTAcTAGATTA GAGCCGTCAATAGATAATACATTTGAGG TATCTTTAGGAGCACTAACAACTcATAGATTA GAGCCGTCAATAGATAATACATTTGAGG | oBK090 mismatch 9 |
| *oBK250* | TATCTTTAGGAGCACTAACAACTAATAGATTc GAGCCGTCAATAGATAATACATTTGAGG | oBK090 mismatch 10 |
| *oBK251* | TATCTTTAGGAGCACTAACAACTAATAGATgA GAGCCGTCAATAGATAATACATTTGAGG | oBK090 mismatch 11 |
| *oBK252* | TATCTTTAGGAGCACTAACAACTAATAGAgTA GAGCCGTCAATAGATAATACATTTGAGG | oBK090 mismatch 12 |
| *oBK253* | TATCTTTAGGAGCACTAACAACTAATAGcTTA GAGCCGTCAATAGATAATACATTTGAGG | oBK090 mismatch 13 |
| *oBK254* | TATCTTTAGGAGCACTAACAACTAATAtATTA GAGCCGTCAATAGATAATACATTTGAGG | oBK090 mismatch 14 |
| *oBK255* | TATCTTTAGGAGCACTAACAACTAATcGATTA GAGCCGTCAATAGATAATACATTTGAGG | oBK090 mismatch 15 |
| *oBK256* | TATCTTTAGGAGCACTAACAACTAAgAGATTA GAGCCGTCAATAGATAATACATTTGAGG | oBK090 mismatch 16 |
| *oBK257* | TATCTTTAGGAGCACTAACAACTAcTAGATTA GAGCCGTCAATAGATAATACATTTGAGG | oBK090 mismatch 17 |
| *oBK258* | TATCTTTAGGAGCACTAACAACTcATAGATTA GAGCCGTCAATAGATAATACATTTGAGG | oBK090 mismatch 18 |
| *oAP030* | CGAGTTGTCGAATTGTTTGTAAAG | forward primer to make dsDNA template from pAP001 (152bp) |
| *oAP039* | T*G*G*T*C*AGTTGGCAAATCAAC | reverse primer to make dsDNA template from pAP001 (* = phosphorothioate) |
| *Amplified Product from pAP001* | TGGTCAGTTGGCAAATCAACAGTTGAAAGGAATTGAGGAAGGTTATCTAAAATATCTT TAGGAGCACTAACAACTAATAGATTAGAGCCGTCAATAGATAATACATTTGAGGATTT AGAAGTATTAGACTTTACAAACAATTCGACAACTCG | |

**Table 2. Sequence identity matrices of pAgo proteins of Bacillales bacterium (BabAgo ), Crenotalea thermophila (CrtAgo), Elioreae tepidiphila (EltAgo), Parabacteroides distasonis (PadAgo) and Maribacter polysiphoniae (MapAgo) (A), and of their respective TIR-APAZ effector proteins (B).**

| A | | | | | | |
|---|---|---|---|---|---|---|
| | EltAgo | PadAgo | MapAgo | BapAgo | CrtAgo | |
| EltAgo | 100 | 39.31 | 39.19 | 40 | 40.2 | |
| PadAgo | 39.31 | 100 | 46.28 | 46.08 | 47.48 | |
| MapAgo | 39.19 | 46.28 | 100 | 79.68 | 81.46 | |
| BabAgo | 40 | 46.08 | 79.68 | 100 | 82.05 | |
| CrtAgo | 40.2 | 47.48 | 81.46 | 82.05 | 100 | |

| B | | | | | | |
|---|---|---|---|---|---|---|
| | | EltTIR-APAZ | PadTIR-APAZ | BapTIR-APAZ | CrtTIR-APAZ | MapTIR-APAZ |
| EltTIR-APAZ | | 100 | 25.62 | 28.41 | 27.52 | 29.53 |
| PadTIR-APAZ | | 25.62 | 100 | 32.03 | 31.8 | 33.64 |
| BabTIR-APAZ | | 28.41 | 32.03 | 100 | 68.44 | 72 |
| CrtTIR-APAZ | | 27.52 | 31.8 | 68.44 | 100 | 72.44 |
| MapTIR-APAZ | | 29.53 | 33.64 | 72 | 72.44 | 100 |

## Claims

1. A detection system for a target nucleic acid molecule, preferably a single stranded nucleic acid molecule, comprising
a short pAgo protein, comprising a MID and a PIWI domain;
an effector protein comprising a Toll-Interleukin1 receptor (TIR)-Analog of PAZ (APAZ) domains or SIR2-APAZ domains;
a guide nucleic acid (gNA) that is able to hybridize to the target nucleic acid molecule; and
a nicotinamide adenine dinucleotide (NAD), or an analogue thereof.

2. The detection system of claim 1, wherein the target nucleic acid molecule is a DNA molecule, preferably a single stranded DNA molecule.

3. The detection system of claim 1 or claim 2, wherein the gNA is a ribonucleic acid molecule.

4. The detection system of any one of the previous claims, wherein the gNA is phosphorylated at its 5'-end.

5. The detection system of any one of the previous claims, wherein the gNA comprises at least 9 nucleotides, preferably 12-40 nucleotides, more preferably 17-21 nucleotides.

6. The detection system of any one of the previous claims, wherein the nicotinamide adenine dinucleotide or analogue thereof is a nicotinamide adenine dinucleotide analogue that becomes fluorescent upon conversion of the nicotinamide adenine dinucleotide, such as ε-NAD⁺ or ε-NADP⁺.

7. The detection system of any one of the previous claims, wherein the short pAgo protein and/or effector protein is from *Bacillales bacterium, Crenotalea thermophila, Elioreae tepidiphila, Maliponia aquimaris, Maribacter polysiphoniae, Mesorhizobium sp., Parabacteroides distasonis, Pannonibacter phragmitetus, Geobacter sulfurreducens, Bradyrhizobium elhanii, Xanthomonas vesicatoria, and*/*or Pseudomonas putida,* preferably originate from *Crenotalea thermophila* or from *Maribacter polysiphoniae.*

8. The detection system of any one of the previous claims, which is a thin layer chromatography or a lateral flow assay.

9. The detection system of any one of the previous claims, wherein the short pAgo protein and TIR-APAZ or SIR2-APAZ effector protein is present as a single protein, for example as a Sir2-APAZ-pAgo protein originating from *Joostella marina, Bacteroides eggerthii, Thioalkalivibrio paradoxus,* and/or *Bacteroides fragilis,* or as a TIR-APAZ-pAgo protein originating from *Rhizobium leguminosarum, Neorhizobium huautlense, Rhizobium viscosum, Labrenzia* sp. EL_126, and/or *Verrucomicrobiaceae bacterium.*

10. A method of detecting a target nucleic acid molecule in a sample comprising nucleic acid molecules, preferably comprising single stranded nucleic acid molecules, the method comprising the steps of:
contacting said sample with a short pAgo protein, comprising a MID and a PIWI domain; an effector protein comprising Toll-Interleukin 1 receptor (TIR)-Analog of PAZ (APAZ) domains or SIR2-APAZ domains, or a combination of a pAgo protein and a TIR-APAZ or SIR2-APAZ effector protein; a guide nucleic acid (gNA) that is able to hybridize to the target nucleic acid molecule; and a nicotinamide adenine dinucleotide or analogue thereof, preferably contacting said sample with the detection system of any one of claims 1-9; and detecting conversion of said nicotinamide adenine dinucleotide or analogue thereof.

11. The method according to claim 10, wherein the sample comprises DNA molecules, preferably single stranded DNA molecules.

12. The method according to claim 10 or 11, wherein the sample comprising nucleic acid molecules has been amplified prior to detecting the nucleic acid molecule in said sample.

13. The method according to claim 12, wherein said amplification is performed by polymerase chain reaction (PCR) or by isothermal amplification, preferably by strand displacement amplification (SDA), nucleic acid sequence-based amplification (NASBA), or loop-mediated isothermal amplification (LAMP).

14. The method according to any one of claims 10-13, wherein the conversion of said nicotinamide adenine dinucleotide or analogue thereof is performed at a temperature between 20 °C and 75 °C, preferably between 50 °C and 65 °C.

15. The method according to any one of claims 10-14, wherein conversion of nicotinamide adenine dinucleotide or analogue thereof is determined by a colorimetric method or a fluorescent method.

## Patentansprüche

1. Detektionssystem für ein Nukleinsäure-Zielmolekül, vorzugsweise ein einsträngiges Nukleinsäuremolekül, umfassend
ein kurzes pAgo-Protein, umfassend eine MID- und eine PIWI-Domäne;
ein Effektorprotein, umfassend ein Toll-Interleukin1-Rezeptor-(TIR)-Analogon von PAZ- (APAZ-) Domänen oder SIR2-APAZ-Domänen;
eine Leitnukleinsäure (gNA), die mit dem Nukleinsäure-Zielmolekül hybridisieren kann; und
ein Nicotinamidadenindinukleotid (NAD) oder ein Analogon davon.

2. Detektionssystem nach Anspruch 1, wobei das Nukleinsäure-Zielmolekül ein DNA-Molekül, vorzugsweise ein einsträngiges DNA-Molekül ist.

3. Detektionssystem nach Anspruch 1 oder Anspruch 2, wobei die gNA ein Ribonukleinsäuremolekül ist.

4. Detektionssystem nach einem der vorhergehenden Ansprüche, wobei die gNA an ihrem 5'-Ende phosphoryliert ist.

5. Detektionssystem nach einem der vorhergehenden Ansprüche, wobei die gNA mindestens 9 Nukleotide, vorzugsweise 12-40 Nukleotide, bevorzugter 17-21 Nukleotide umfasst.

6. Detektionssystem nach einem der vorhergehenden Ansprüche, wobei das Nicotinamidadenindinukleotid oder ein Analogon davon ein Nicotinamidadenindinukleotid-Analogon ist, das bei Umwandlung des Nicotinamidadenindinukleotids fluoreszierend wird, wie ε-NAD⁺ oder ε-NADP⁺.

7. Detektionssystem nach einem der vorhergehenden Ansprüche, wobei das kurze pAgo-Protein und/oder Effektor-Protein vom *Bacillales-Bakterium, Crenotalea thermophila, Elioreae tepidiphila, Maliponia aquimaris, Maribacter polysiphoniae, Mesorhizobium sp., Parabacteroides distasonis, Pannonibacter phragmitetus, Geobacter sulfurreducens, Bradyrhizobium elkanii, Xanthomonas vesicatoria, und*/*oder Pseudomonas putida* sind, vorzugsweise vom *Crenotalea thermophila* oder vom *Maribacter polysiphoniae* stammen.

8. Detektionssystem nach einem der vorhergehenden Ansprüche, das eine Dünnschichtchromatographie oder ein Seitenflusstest ist.

9. Detektionssystem nach einem der vorhergehenden Ansprüche, wobei das kurze pAgo-Protein und das TIR-APAZ- oder SIR2-APAZ-Effektor-Protein als ein einzelnes Protein vorhanden sind, beispielsweise als ein Sir2-APAZ-pAgo-Protein, das von *Joostella marina, Bacteroides eggerthii, Thioalkalivibrio paradoxus* und/oder *Bacteroides fragilis* stammt, oder als ein TIR-APAZ-pAgo-Protein, das von *Rhizobium leguminosarum, Neorhizobium huautlense, Rhizobium viscosum, Labrenzia* Sp. EL_126 stammt, und/oder ein Bakterium der *Verrucomicrobiaceae.*

10. Verfahren zum Detektieren eines Nukleinsäure-Zielmoleküls in einer Probe, umfassend Nukleinsäuremoleküle, vorzugsweise umfassend einsträngige Nukleinsäuremoleküle, das Verfahren umfassend die Schritte von:
Kontaktieren der Probe mit einem kurzen pAgo-Protein, umfassend eine MID- und eine PIWI-Domäne; einem Effektor-Protein, umfassend ein Toll-Interleukin-1-Rezeptor-(TIR-)Analogon von PAZ-(APAZ-) Domänen oder SIR2-APAZ-Domänen, oder eine Kombination aus einem pAgo-Protein und einem TIR-APAZ- oder SIR2-APAZ-Effektor-Protein; einer Leitnukleinsäure (gNA), die mit dem Nukleinsäure-Zielmolekül hybridisieren kann; und einem Nicotinamidadenindinukleotid oder einem Analogon davon, vorzugsweise Kontaktieren der Probe mit dem Detektionssystem nach einem der Ansprüche 1 bis 9; und Detektieren der Umwandlung des Nicotinamidadenindinukleotids oder eines Analogons davon.

11. Verfahren nach Anspruch 10, wobei die Probe DNA-Moleküle, vorzugsweise einsträngige DNA-Moleküle, umfasst.

12. Verfahren nach Anspruch 10 oder 11, wobei die Probe, umfassend Nukleinsäuremoleküle, vor dem Detektieren des Nukleinsäuremoleküls in der Probe amplifiziert worden ist.

13. Verfahren nach Anspruch 12, wobei die Amplifikation durch Polymerasekettenreaktion (PCR) oder durch isotherme Amplifikation, vorzugsweise durch Strangverdrängungsamplifikation (SDA), nukleinsäuresequenzbasierte Amplifikation (NASBA) oder schleifenvermittelte isotherme Amplifikation (LAMP) durchgeführt wird.

14. Verfahren nach einem der Ansprüche 10 bis 13, wobei die Umwandlung des Nicotinamidadenindinukleotids oder eines Analogons davon bei einer Temperatur zwischen 20 °C und 75 °C, vorzugsweise zwischen 50 °C und 65 °C, durchgeführt wird.

15. Verfahren nach einem der Ansprüche 10 bis 14, wobei die Umwandlung von Nicotinamidadenindinukleotid oder eines Analogons davon durch ein kolorimetrisches Verfahren oder ein Fluoreszenzverfahren bestimmt wird.

## Revendications

1. Système de détection pour une molécule d'acide nucléique cible, de préférence une molécule d'acide nucléique simple brin, comprenant une protéine pAgo courte, comprenant
un domaine MID et un domaine PIWI ;
une protéine effectrice comprenant un analogue du récepteur Toll-Interleukine1 (TIR) des domaines PAZ (APAZ) ou des domaines SIR2-APAZ ;
un acide nucléique guide (ANg) qui est capable de s'hybrider à la molécule d'acide nucléique cible ; et
un nicotinamide adénine dinucléotide (NAD), ou un analogue de celui-ci.

2. Système de détection selon la revendication 1, dans lequel la molécule d'acide nucléique cible est une molécule d'ADN, de préférence une molécule d'ADN simple brin.

3. Système de détection selon la revendication 1 ou la revendication 2, dans lequel l'ANg est une molécule d'acide ribonucléique.

4. Système de détection selon l'une quelconque des revendications précédentes, dans lequel l'ANg est phosphorylé au niveau de son extrémité 5'.

5. Système de détection selon l'une quelconque des revendications précédentes, dans lequel l'ANg comprend au moins 9 nucléotides, de préférence de 12 à 40 nucléotides, de préférence encore de 17 à 21 nucléotides.

6. Système de détection selon l'une quelconque des revendications précédentes, dans lequel le nicotinamide adénine dinucléotide ou un analogue de celui-ci est un analogue de nicotinamide adénine dinucléotide qui devient fluorescent lors de la conversion du nicotinamide adénine dinucléotide, tel que ε-NAD⁺ or ε-NADP⁺.

7. Système de détection selon l'une quelconque des revendications précédentes, dans lequel la protéine pAgo courte et/ou la protéine effectrice provient de *Bacillales bacterium, Crenotalea thermophila, Elioreae tepidiphila, Maliponia aquimaris, Maribacter polysiphoniae, Mesorhizobium sp., Parabacteroides distasonis, Pannonibacter phragmitetus, Geobacter sulfurreducens, Bradyrhizobium elkanii, Xanthomonas vesicatoria,* et/ou *dePseudomonas putida,* provient de préférence de *Crenotalea thermophila* ou de *Maribacter polysiphoniae.*

8. Système de détection selon l'une quelconque des revendications précédentes, qui est une chromatographie sur couche mince ou un test à flux latéral.

9. Système de détection selon l'une quelconque des revendications précédentes, dans lequel la protéine pAgo courte et la protéine effectrice TIR-APAZ ou SIR2-APAZ sont présentes sous forme d'une seule protéine, par exemple sous forme d'une protéine Sir2-APAZ-pAgo provenant de *Joostella marina, Bacteroides eggerthii, Thioalkalivibrio paradoxus* et/ou *Bacteroides fragilis,* ou sous forme d'une protéine TIR-APAZ-pAgo provenant de *Rhizobium leguminosarum, Neorhizobium huautlense, Rhizobium viscosum, Labrenzia sp.EL_126,* et/ou de *Verrucomicrobiaceae bacterium.*

10. Procédé de détection d'une molécule d'acide nucléique cible dans un échantillon comprenant des molécules d'acide nucléique, comprenant de préférence des molécules d'acide nucléique simple brin, le procédé comprenant les étapes consistant à :
mettre en contact ledit échantillon avec une protéine pAgo courte, comprenant un domaine MID et un domaine PIWI ; une protéine effectrice comprenant un analogue du récepteur Toll-Interleukine 1 (TIR) de domaines PAZ (APAZ) ou de domaines SIR2-APAZ, ou une combinaison d'une protéine pAgo et d'une protéine effectrice TIR-APAZ ou SIR2-APAZ ; un acide nucléique guide (ANg) qui est capable de s'hybrider à la molécule d'acide nucléique cible ; et un nicotinamide adénine dinucléotide ou un analogue de celui-ci, de préférence mettre en contact ledit échantillon avec le système de détection selon l'une quelconque des revendications 1 à 9 ; et détecter la conversion dudit nicotinamide adénine dinucléotide ou d'un analogue de celui-ci.

11. Procédé selon la revendication 10, dans lequel l'échantillon comprend des molécules d'ADN, de préférence des molécules d'ADN simple brin.

12. Procédé selon la revendication 10 ou 11, dans lequel l'échantillon comprenant des molécules d'acide nucléique a été amplifié avant la détection de la molécule d'acide nucléique dans ledit échantillon.

13. Procédé selon la revendication 12, dans lequel ladite amplification est effectuée par réaction en chaîne de la polymérase (PCR) ou par amplification isotherme, de préférence par amplification par déplacement de brin (SDA), amplification d'une séquence ADN (NASBA), ou amplification isotherme à médiation par boucle (LAMP).

14. Procédé selon l'une quelconque des revendications 10 à 13, dans lequel la conversion dudit nicotinamide adénine dinucléotide ou de son analogue est effectuée à une température comprise entre 20 °C et 75 °C, de préférence entre 50 °C et 65 °C.

15. Procédé selon l'une quelconque des revendications 10 à 14, dans lequel la conversion du nicotinamide adénine dinucléotide ou d'un analogue de celui-ci est déterminée par une méthode colorimétrique ou une méthode de fluorescence.
